(19) Europäisches Patentamt
European Patent Office
Office européen des brevets

(11) Publication number: 0 384 636 A1

(12) # EUROPEAN PATENT APPLICATION

(21) Application number: 90301579.0

(22) Date of filing: 14.02.90

(51) Int. Cl.5: C07C 327/04, A61K 31/265, C07C 323/60, A61K 31/095, C07D 207/09, C07D 277/04, C07F 9/09

(30) Priority: 21.02.89 GB 8903887
21.02.89 GB 8903888
21.02.89 GB 8903889
02.11.89 GB 8924672
02.11.89 GB 8924673
02.11.89 GB 8924674

(43) Date of publication of application: 29.08.90 Bulletin 90/35

(84) Designated Contracting States: AT BE CH DE DK ES FR GB GR IT LI LU NL SE

(71) Applicant: FISONS plc
Fison House Princes Street
Ipswich Suffolk IP1 1QH(GB)

(72) Inventor: Baxter, Andrew John Gilby
Flat 4, 6 Western Terrace, The Park
Nottingham(GB)
Inventor: Robinson, David Hulme
12 Grassholme Drive
Loughborough, Leicestershire(GB)
Inventor: Brown, Roger Charles
8 Duncan Way, Gorse Covert
Loughborough, Leicestershire(GB)

(74) Representative: Wright, Robert Gordon McRae
FISONS plc 12 Derby Road
Loughborough Leicestershire LE11 0BB(GB)

(54) **Pharmaceutically active compounds.**

(57) There are described compounds of formula I, processes for their preparation and pharmaceutical formulations containing them,

$ZCHR_1COR_2$ I

in which $R_2$ is a group of formula II,

$-NR_xCHR_yCOOH$ II

in which $R_x$ and $R_y$ are each alkyl optionally substituted by phenyl; phenylalkyl C7 to 12, phenyl, phenyloxyalkyl, N-alkyl, O-alkyl, or together $R_x$ and $R_y$ may form a heterocyclic group optionally substituted by alkyl, phenyl; phenylalkyl C7 to 12, phenyl, phenyloxyalkyl, N-alkyl, O-alkyl, or one of $R_x$ and $R_y$ may be hydrogen, provided that one of $R_x$ and $R_y$, the heterocyclic group formed by $R_x$ and $R_y$ or the optional substituents is substituted by one or two groups $-COOR_9$

$R_9$ is phenyl, $-(CH_2)_xCONR_{14}R_{15}$, $-(CH_2)_yCOOR_{16}$ or alkyl optionally substituted by phenyl;

$R_{14}$, $R_{15}$, $R_{16}$, x and y, are as described in the specification,

and pharmaceutically acceptable salts thereof.

EP 0 384 636 A1

## Pharmaceutically Active Compounds

This invention relates to new compounds, methods for their preparation and compositions containing them.

A wide variety of angiotensin converting enzyme (ACE) inhibitors are known, eg from European Patent Application No. 0217519 and South African Patent Application No. 834454.

Such known ACE inhibitors have a relatively long duration of activity. However, we have now found a new group of compounds which have advantageous properties as ACE inhibitors. In particular, these compounds possess a relatively short duration of activity thus indicating them in the treatment of critically ill patients.

According to the invention we provide compounds of formula I,

$ZCHR_1COR_2$ I

in which $R_2$ is a group of formula II,

$-NR_xCHR_yCOOH$ II

in which $R_x$ and $R_y$, which may be the same or different, are each cycloalkyl C3 to 12, straight chain or branched chain alkyl C 1 to 6 optionally substituted by phenyl; phenylalkyl C7 to 12, phenyl, phenyloxyalkyl, N-alkyl, O-alkyl, or together $R_x$ and $R_y$ may form a heterocyclic group optionally substituted by cycloalkyl C 3 to 12, straight chain or branched chain alkyl C 1 to 6 phenyl; phenylalkyl C7 to 12, phenyl, phenyloxyalkyl, N-alkyl, O-alkyl, or one of $R_x$ and $R_y$ may be hydrogen,

provided that one of $R_x$ and $R_y$, the heterocyclic group formed by $R_x$ and $R_y$ or the optional substituents is substituted by one or two groups $-COOR_9$

$R_9$ is phenyl, $-(CH_2)_xCONR_{14}R_{15}$, $-(CH_2)_yCOOR_{16}$ or straight, branched or cyclic alkyl C 1 to 12 optionally substituted by phenyl;

Z is $R_{20}$ $CH(COOH)NR_{21}$, $R_{26}SCH_2$-, $R_{22}$ $\underset{\substack{\| \\ O}}{P}$ (OH)-,

or $R_{23}CONR_{24}CHR_{25}$ $CO(CH_2)_z$-,

$R_1$, $R_{14}$, $R_{15}$, $R_{21}$ and $R_{24}$, which may be the same or different, are each hydrogen or alkyl C 1 to 6,

$R_{16}$ is alkyl C 1 to 6,

$R_{20}$, $R_{22}$, $R_{23}$ and $R_{25}$, which may be the same or different, are each phenyl or phenylalkyl C 7 to 12,

$R_{26}$ is hydrogen or alkanoyl C2 to 7,

x, y and z, which may be the same or different, are each an integer from 1 to 6,

and pharmaceutically acceptable salts thereof.

According to the invention we also provide a process for the production of a compound of formula I, or a pharmaceutically acceptable salt thereof which comprises

a) removal of a protecting group from a compound of formula I in which one or more of the amino, thiol or carboxylic acid groups is protected,

b) reacting a compound of formula III, or a salt or ester thereof,

$ZCHR_1COL_b$ III

in which Z and $R_1$ are as defined above, and

$L_b$ is a good leaving group

with a compound of formula IV, or a salt thereof,

$HNR_xCHR_yCOOH$ IV

in which $R_x$ and $R_y$ are as defined above,

c) production of a pharmaceutically acceptable salt of a compound of formula I, by treating a compound of formula I, or another salt thereof, with a compound containing an available pharmaceutically acceptable ion and capable of converting the compound of formula I or the other salt thereof, to a pharmaceutically acceptable salt of the compound of formula I, and where desired or necessary deprotecting the resulting compound, or converting a compound of formula I to a pharmaceutically acceptable salt thereof or vice versa.

In process a) the protecting group can be any convenient protecting group conventionally used in peptide synthesis and may be removed using techniques conventionally used in peptide synthesis. Thus protecting groups which may be used are alkoxy C 1 to 6, which may be a straight chain or branched alkoxy, eg t-butyloxy; phenylalkoxy C 7 to 12, eg benzyloxy; or alkanoyl C 2 to 7, eg acetyl. These groups can be removed by hydrolysis, for example basic hydrolysis, eg using ageous methanolic sodium or potassium hydroxide; or cleavage using, for example, trifluoroacetic acid; or by hydrogenation, eg using palladium on charcoal. Amino-protecting groups which may be mentioned include alkyloxycarbonyl C 2 to 7, eg t-butyloxycarbonyl, or phenylalkyloxycarbonyl C 8 to 13, eg benzyloxycarbonyl.

In process b) the good leaving group $L_b$ may be, for example, halogen and the reaction may be carried out in an inert solvent, eg dichloromethane at a temperature of from about 0° to 100°. When $L_b$ = halogen the reaction may be carried out in the presence of base, eg pyridine.

The reaction may comprise the formation of, optionally in situ, an activated derivative of an acid, eg an anhydride or dicyclohexylcarbodiimide derivative. The reaction may be carried out in a solvent which is inert under the reaction conditions, eg dichloromethane or ethyl acetate, at a temperature of from -10°C to the boiling point of the solvent, preferably from 0°C to 30°C. The reaction may be carried out in the presence of a base, eg triethylamine. When the reaction involves dicyclohexylcarbodiimide it may be carried out in the presence of an activating agent, eg hydroxybenzotriazole.

The reaction will of course vary with the particular activated derivative used.

In process c) the salts may be formed by reacting the free acid, or a salt thereof, or the free base, or a salt or derivative thereof, with one or more equivalents of the appropriate base or acid. The reaction may be carried out in a solvent or medium in which the salt is insoluble or in a solvent in which the salt is soluble, eg ethanol, tetrahydrofuran or diethyl ether, which may be removed in vacuo, or by freeze drying. The reaction may also be a metathetical process or it may be carried out on an ion exchange resin.

Pharmaceutically acceptable salts of the compounds of formula I include ammonium salts, alkali metal salts, eg sodium and potassium salts; alkaline earth metal salts, eg the calcium and magnesium salts; salts with organic bases, eg N-methyl-D-glucamine; and salts with amino acids, eg with arginine, lysine etc. Also, when the molecule contains a basic group, salts with organic or inorganic acids, eg with HCl, HBr, $H_2SO_4$, methanesulfonic, toluenesulfonic, maleic, fumaric or camphorsulfonic acids. The non-toxic physiologically acceptable salts are preferred, although other salts are also useful, eg in isolating or purifying the product.

Preferred organic bases included dicyclohexylamine and adamantanamine.

Preferred organic acids include trifluoroacetic acid.

The compounds of formula III are either known or may be made by conventional processes known per se.

The compounds of formula IV may be prepared by reacting a compound of formula XXVIII, or an ester thereof,

$L_cCHR_yCOOH$ XXVIII

in which $R_y$ is as defined above, and

$L_c$ is a leaving group

with a compound of formula XXIV,

$R_xNH_2$ XXIV

in which $R_x$ is as defined above,

or when $R_x$ and $R_y$ together form a heterocyclic group, by reacting a suitable heterocycle eg.a compound of formula XXX, or an ester or a carbamate thereof,

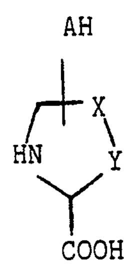

XXX

in which X and Y are as defined above, and

A is -O- or a bond,

with a compound of formula XXXI,

$L_x(CH_2)_mCOOR_{13}$ XXXI

in which m and $R_{13}$ are as defined above, and

$L_x$ is a leaving group or a displaceable group, eg. diazo.

The starting materials for all of the above processes are either known or may be made from compounds using conventional processes known per se.

The compounds of formula I, and the intermediates thereof, may be isolated from their reaction mixtures using conventional techniques known per se.

The processes described above may produce the compound of formula I or a derivative thereof. It is also within the scope of this invention to treat any derivative so produced to liberate the free compound of formula I, or convert one derivative into another.

In addition to the processes described above the compounds of formula I may be made by a variety of processes which are analogous to those known for the production of structurally similar compounds.

By the term alkyl we mean straight, branched or cyclic alkyl groups.

The compounds of formula I may contain one or more asymmetric carbon atoms and may therefore exhibit optical and/or diastereo isomerism. Diastereoisomers may be separated using conventional techniques, eg chromatography or fractional crystallisation. The various optical isomers may be isolated by separation of a racemic or other mixture of the compounds using conventional, eg fractional crystallisation or HPLC, techniques. Alternatively the desired optical isomers may be made by reaction of the appropriate optically active starting materials under conditions which will not cause racemisation.

When $R_x$ and $R_y$ together form a heterocyclic group, we prefer this to be a 5-membered heterocyclic group.

Preferred groups of $R_2$ are those in which $R_x$ is a group $R_3$ or $R_5$ and $R_y$ is a group $R_4$ or $(CH_2)_nCOOR_6$
or in which $R_x$ and $R_y$ together form a group of formula XXVII

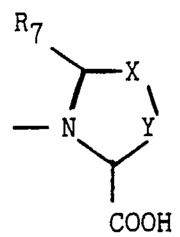

XXVII

in which one of X and Y is $-CHR_8$ or S and the remainder is $-CH_2-$
$R_3$ is cycloalkyl C 3 to 12, straight chain or branched chain alkyl C 1 to 6 optionally substituted by phenyl; phenylalkyl C7 to 12, phenyl, phenyloxyalkyl, N-alkyl or O-alkyl; each of which is substituted by one or two groups $-COOR_9$,
$R_5$ is hydrogen, cycloalkyl C3 to 12 or straight chain or branched chain alkyl C 1 to 7
$R_6$ is alkyl C 1 to 6, $-(CH_2)_vCOOR_{10}$ or $(CH_2)_wCONR_{11}R_{12}$,
one of $R_7$ and $R_8$ is $-A(CH_2)_mCOOR_{13}$ and the remainder is hydrogen or phenyl,
$R_{13}$ is alkyl C 1 to 6, $-CH_2COOR_{17}$ or $CH_2CONR_{18}R_{19}$,
$R_4$, $R_{11}$, $R_{12}$, $R_{18}$ and $R_{19}$, which may be the same or different, are each hydrogen or alkyl C 1 to 6,
$R_{10}$ and $R_{17}$, which may be the same or different, are each alkyl C 1 to 6,
n, v and w which may be the same or different, are each an integer from 1 to 6,
m is an integer from 0 to 6, and
A and $R_9$ are as defined above.

We prefer compounds in which Z is $R_{20}CH(COOH)NR_{21}$, or more preferably $R_{26}SCH_2$. $R_1$ is preferably hydrogen or more preferably methyl.

When $R_2$ is a heterocyclic ring it is preferably a pyrrolidine-2-carboxylic acid, more preferably a 5-substituted pyrrolidine-2-carboxylic acid.

$R_3$ is preferably phenyl or alkyl. When $R_3$ is phenylalkyl the group $-COOR_9$ is preferably positioned on the alkyl group. Such alkyl groups may be ortho or meta but are preferably para substituted. When $R_3$ is straight chain alkyl it may be methyl, ethyl or propyl, or $R_3$ may branched alkyl eg 1-methylethyl or 1,1 dimethylethyl. We prefer $R_3$ to be substituted by only one $-COOR_9$ group.

$R_4$ and $R_5$ are preferably alkyl C 1 to 2, eg. methyl or more preferably hydrogen.

Any one of $R_6$, $R_{11}$, $R_{12}$, $R_{13}$, $R_{14}$, $R_{15}$, $R_{18}$ and $R_{19}$ are preferably alkyl C 1 to 6, more preferably alkyl C 1 to 4 and especially methyl.

We prefer the remainder of $R_7$ and $R_8$ which is not $-A(CH_2)_mCOOR_{13}$ to be hydrogen. A is preferably 0 and m is preferably an integer from 0 to 4, more preferably 1. $R_{13}$ is preferably alkyl C 1 to 6, more preferably alkyl C 1 to 4, eg methyl or ethyl. When m is 0 we prefer $R_{13}$ to be methyl, when m is 1 we prefer $R_{13}$ to be ethyl.

$R_9$ is preferably cycloalkyl C 3 to 12, straight chain or branched chain alkyl C 1 to 6, preferably alkyl C 1 to 4, alkylphenyl C7 to 12, phenyl or $-(CH_2)_xCONR_{14}R_{15}$. When $R_9$ is $-(CH_2)_xCONR_{14}R_{15}$, x is preferably 1-2 or more preferably 1, $R_{14}$ and $R_{15}$ are both preferably alkyl C 1 to 6 eg methyl. When $R_9$ is alkylphenyl it is preferably benzyl. $R_9$ is more preferably straight, branched or cyclic alkyl. The preferred cycloalkyl group is a $C_4$-$C_6$ cycloalkyl group, eg cyclohexane. Such cycloalkyl groups may be attached to an alkyl chain of $R_9$, eg $CH_2$-cycloaklyl or $CH_2CH_2$-cycloalkyl. Branched alkyl groups include 1-methylethyl, 1,1-dimethylethyl and especially 2-methylpropyl. The most preferred group of $R_9$ is straight chain alkyl C 1 to 6, more preferably ethyl and especially methyl.

Any one of $R_{10}$, $R_{16}$ and $R_{17}$ are preferably alkyl C 1 to 4, eg ethyl and especially methyl.

$R_{20}$ is preferably phenylalkyl C7 to 12, the alkyl chain preferably comprises 1 to 3 carbon atoms. $R_{20}$ is most preferably phenylethyl.

Any one of $R_{21}$ and $R_{24}$ are preferably hydrogen.

$R_{22}$ is preferably phenylalkyl C7 to 12, the alkyl chain preferably comprises from 2 to 4 carbon atoms. $R_{22}$ is most preferably phenylpropyl.

$R_{23}$ is preferably phenyl.

$R_{25}$ is preferably phenylalkyl C7 to 12, the alkyl chain preferably comprises 1 to 3 carbon atoms. $R_{25}$ is most preferably benzyl.

$R_{26}$ is preferably alkanoyl and more preferably acetyl.

A number of preferred groupings may be mentioned. Preferred groups for $ZCHR_1CO-$ include, $CH_3COSCH_2CH(CH_3)CO-$, $PhCONHCH(CH_2Ph)COCH_2CH_2CO$ and $PhCH_2CH_2CH(COOH)NHCH(CH_3)CO-$ . The most preferred group is $HSCH_2CH(CH_3)CO-$.

When $R_2$ is a heterocyclic group groups which may be mentioned include 2-pyrrolidine carboxylic acid derivatives such as the 5-carboxymethylester, 4-carboxymethylester-5-phenyl, 4-carboxymethylester, 5-acetic acid methyl ester and 4-(2-ethoxy-2-oxoethoxy). Thus a preferred group of compounds are those of the general formula XX

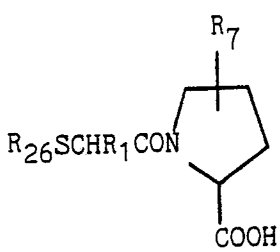

XX

in which $R_1$, $R_7$ and $R_{26}$ are as defined above.

$R_7$ is preferably in the 4- or 5- position.

A further group which may be mentioned are the thiazole-2-carboxylic acid derivatives such as 2,4-thiazolidine diacid 2-methyl ester and 2,4-thiazolidine diacid 4-methyl ester.

A further group of compounds which may be mentioned is the group of formula XXI

$ZCHR_1CON(CH_2COOH)(CH_2)_tCOOR_9$ XXI

in which Z, $R_1$ and $R_9$ are as defined above, and

t is an integer from 1 to 6.

A preferred group of such compounds are those in which Z is $R_{26}S$ and especially prefered are those compounds in which $R_{26}$ is hydrogen.

A further group of compounds which may be mentioned are those of general formula XXII.

$ZCHR_1CON(CH_2COOH)CH(COOR_9)R_{27}$ XXII

in which Z, $R_1$ and $R_9$ are as defined above, and

$R_{27}$ is cycloalkyl C 3 to 12, straight chain or branched chain alkyl C 1 to 6, preferably alkyl C 1 to 4.

The compounds of formula IV are useful as intermediates for the preparation of compounds of formula I.

Thus according to the invention we provide compounds of formula IV, or a salt or protected derivatives thereof,

$HNR_xCHR_yCOOH$ IV

in which $R_x$ and $R_y$ are as defined above,

By the term protected derivatives we mean compounds protected by protecting groups hereinbefore described.

We prefer compounds of formula IV in which $-NR_xCHR_yCOOH$ is a group $-NR_3CHR_4COOH$ or $-NR_5CH(COOH)(CH_2)_nCOOR_6$.

The compounds of formula I in which the COOH group is protected are also useful as intermediates for the preparation of compounds of formula I.

Thus according to the invention we provide compounds of formula XXIII.

$ZCHR_1COR_{2a}$ XXIII

in which Z and $R_1$ are as defined above, and

$R_{2_a}$ is a group of formula XXIV, XXV or XXVII

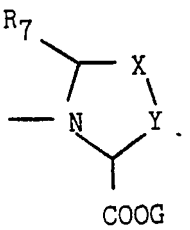

XXIV

$-NR_3CHR_4COOG$ XXV

$-NR_5CH(COOG)(CH_2)_nCOOR_6$ XXVII

in which $R_3$, $R_4$, $R_5$, $R_6$, $R_7$ and n are as defined above, and G is a protecting group hereinbefore described.

The compounds of formula I in which Z is $R_{26}SCH_2$-are useful as intermediates in the preparation of compounds of formula I in which Z is $HSCH_2$-.

We further provide compounds of formula XXVI

$R_{26}SCH_2CHR_1COR_2$ XXVI

in which $R_{26}$, $R_1$ and $R_2$ are as defined above, for use as intermediates.

The compounds of the invention are advantageous in that they are more efficaceous, produce less side effects, are shorter acting, more readily absorbed, less toxic, distributed in the body tissues in a different manner or have other advantageous properties when compared to compounds of similar structure.

The compounds of the invention are useful because they possess pharmacological properties. In particular they inhibit angiotensin converting enzyme and thus block conversion of the decapeptide angiotensin I to angiotensin II (see Example X). Angiotensin II is a potent vasoconstrictor in mammals. Angiotensin II stimulates aldosterone release which results in salt and fluid retention, it is pro-arrhythmic and exacerbates damage due to myocardial infarction. Inhibitors of angiotensin converting enzyme are thus effective vasodilators in a variety of animal models (see Example Y) and are indicated for use clinically, for example, in patients with acute myocardial infarction, acute heart failure or hypertension. See, for example, D W Cushman et al Biochemistry 16, 5484 (1977); E W Petrillo and M A Ondetti, Med. Res. Rev. 2 93 (1982) or H M McAlpine, J J Morton; B Leckie and H J Dargie J. Cardiavas. Pharmacol. 9 (Suppl 2) S25-S30 (1987).

Thus, the compounds of the invention are useful as vasodilators in treating heart failure, renal failure, hypertension, angina pectoris and ischaemic heart disease in mammals, including humans and they can be utilised to achieve vasodilation, eg in formulations containing appropriate pharmaceutically acceptable excipients, diluents or carriers. The compounds of the invention can be administered (to animals or humans) by a variety of routes, eg sublingually or intramuscularly and especially intravenously at dosages of 1 to 5mg generally over several hours, to a total daily dose of 10mg per day. The dose will vary depending on the type and severity of disease, weight of patient and other factors which a person skilled in the art will recognise.

Thus according to the invention we provide the first pharmaceutical use of a compound of formula I. We provide a compound of formula I for use as a medicament. In particular we provide the use of a compound of formula I in the manufacture of a medicament for the treatment of heart failure. We further provide the use of a compound of formula I in the manufacture of a medicament for the treatment of any of the following conditions; renal failure, hypertension, angina pectoris and ischaemic heart disease.

The compounds of this invention may be given in combination with other pharmaceutically active compounds, eg diuretics, thrombolytics or antihypertensives. The dosage of the other pharmaceutically active compound can be that conventionally used when the compound is administered on its own, but is preferably somewhat lower. To illustrate these combinations, one of the vasodilators of this invention can be combined at levels ranging, eg from 1-200 milligrams per day with the following antihypertensives and diuretics in dose ranges per day as indicated:

hydrochlorothiazide (15-200mg), chlorothiazide (125-2000mg), ethacrynic acid (15-200mg), amiloride (5-20mg), furosemide (5-600mg), atenolol (5-100mg), propanolol (20-640mg), verapamil (120-480mg) and methyldopa (65-2000mg). In addition, the triple drug combinations of hydrochlorothiazide (15-200mg) plus amiloride (5-20mg) plus converting enzyme inhibitor of this invention (1-200mg) or hydrochlorothiazide (15-200mg) plus timolol (5-50mg), plus the converting enzyme inhibitor of this invention (1-200mg) are

contemplated. The following compounds may also be combined; inotropes, eg amrinone (5µg-600mg; enoximone (5µg-600mg); vasodilators, eg nitroglycerine (1-15mg), isosorbide (1-150mg), sodium nitroprusside (1-600mg); inotropic vasodilators, eg dopamine (1-50mg), dobutamine (1-50mg); osmotic diuretics, eg mannitol (50-200g); antiarrhythmics, eg procainamide (1-1000mg); thrombolytics, eg streptokinase (100,000-600,000 IU); opiode analgesics, eg butorphanol, codeine, diamorphine (1-5mg), methadone; antibiotics, eg sulphonamide, tetracyclines, penicillins; analgesics, eg aspirin (1mg-1g), electrolytes, eg sodium, potassium, magnesium; acids and bases, eg sodium bicarbonate, ammonium chloride, citric acid, sodium citrate. The above dose ranges may be adjusted on a unit basis as necessary to permit divided daily dosage. Also, the dose may vary depending on the severity of the disease, weight of patient and other factors which a person skilled in the art will recognise.

According to our invention we also provide a pharmaceutical composition comprising preferably less than 80%, more preferably less than 50%, eg 1 to 20%, by weight of a compound of formula I, or a pharmaceutically acceptable salt thereof, in admixture with a pharmaceutically acceptable adjuvant, diluent or carrier.

Thus the compound may be put up as a tablet, capsule, dragee, suppository, suspension, solution, injection, eg, intravenously, intramuscularly or intraperitoneally, implant, a topical, eg transdermal, preparation such as a gel, cream, ointment, aerosol or a polymer system, or an inhalation form, eg an aerosol or a powder formulation.

We prefer compositions which are designed to be taken intravenously as a continuous infusion or bolus injection. Thus we prefer a solution which may, for example be made by dissolution in an appropriate vehicle.

Certain of the compounds of formula I can form hydrates or solvates, eg with an alcohol such as ethanol.

The invention will now be illustrated by the following Examples in which temperatures are in degrees celcius.

## Example 1

### N-(Carboxymethyl)-N-(3-mercapto-2(S)-methyl-1-oxo propyl)-β-alanine 1-methyl ester dicyclohexylamine salt.

#### (a) N-(2-(1,1-Dimethylethoxy)-2-oxoethyl)-β-alanine methyl ester

1,1-Dimethylethyl bromoacetate (7.0g, 36mmoles) was added dropwise to a stirred suspension at 0° of β-alanine methyl ester hydrochloride (5.0g, 36mmoles) and triethylamine (7.5g, 74mmoles) in acetonitrile (50ml). After 24 hours, the solvent was evaporated and the residue dissolved in dichloromethane and saturated sodium bicarbonate. The organic layer was separated, dried ($Na_2SO_4$) and the solvent was evaporated.
Chromatography on silica eluting with ethylacetate/petroleum ether (60-80°) mixtures gave the title compound (5.0g).
nmr δ($CDCl_3$) 1.47 (9H,s), 3.31 (2H,s) 3.69 (3H,s).

#### (b) N-(3-(Acetylthio)-2(S)-methyl-1-oxopropyl)-N-(2-(1,1-dimethylethoxy)-2-oxoethyl)-β-alanine methyl ester.

(R)-3-(Acetylthio)-2-methylpropanoyl chloride (1.98g, 111mmoles) was added to a stirred mixture of the product of Example 1a) (2.17g, 10mmoles), polyvinylpyridine (2.5g) and 4-(d imethylamino)pyridine (catalytic quantity) in dichloromethane (50ml). After 16 hours, the solution was filtered; the filtrant being washed with dichloromethane. The combined organic filtrate was washed with 0.5M hydrochloric acid and saturated sodium bicarbonate, dried ($Na_2SO_4$) and the solvent was evaporated.
Chromatography on silica eluting with ethylacetate/petroleum ether (60-80°) mixtures gave the title compound (3.6g).
$M^+$,361, nmr δ($CDCl_3$) 1.46 & 1.47 (9H, s(x2)), 2.31 & 2.34 (3H,s(x2)), 3.67 & 3.70 (3H,s(x2))

(c) N-(3-(Acetylthio)-2(S)-methyl-1-oxopropyl)-N-(carboxymethyl)-β-alanine 1-methyl ester.

Trifluoroacetic acid (2ml) was added to the product of Example 1b) (1.35g, 37mmoles) in dichloromethane (7ml). After stirring for 24 hours, the solvent was evaporated and the residue azeotroped with toluene three times.
Chromatography on silica eluting with dichloromethane/ethylacetate/acetic acid gave the title compound (0.7g).
$M+1^+$,306; nmr δ($CDCl_3$) 2.32 & 2.34 (3H,s(x2)), 3.67 & 3.70 (3H,s(x2)).

(d) N-(Carboxymethyl)-N-(3-mercapto-2(S)-methyl-1-oxopropyl)-β-alanine 1-methyl ester dicyclohexylamine salt.

Potassium hydroxide (0.23g of 85%, 4.1mmoles) in methanol (8ml) was added to a stirred solution under nitrogen of the product of Example 1c) (0.7g, 2.05mmoles) in methanol (20ml). After 2 hours, the solvent was evaporated and the residue dissolved in 2M hydrochloric acid and ethyl acetate. The organic layer was separated, washed with saturated brine, dried ($Na_2SO_4$) and the solvent was evaporated. Chromatography on silica eluting with ethyl acetate/acetic acid followed by salt formulation with dicyclohexylamine (0.36g, 2 mmoles) and trituration with diethylether gave the title compound (0.82g) mp 147-8° $[\alpha]_{23}$ - 14.3° C = 0.21 ($CHCl_3$).

## Example 2

N-(3-Mercapto-2(S)-methyl-1-oxopropyl)-N-(2-methoxy-2-oxoethyl)glycine dicyclohexylamine salt.

Prepared by the method of Example 1 mp 137-8°

## Example 3

3-((Carboxymethyl)(3-mercapto-2(S)-methyl-1-oxopropyl)amino)benzoic acid 1-methyl ester

a) 3-((2-(1,1-Dimethylethoxy)-2-oxoethyl)amino)benzoic acid methyl ester

Methyl 3-aminobenzoate (5g, 33mmoles), 1,1-dimethylethyl bromoacetate (6.5g, 33mmoles) and potassium carbonate (9g, 66mmoles) in dimethylformamide (35ml) were heated with stirring at 50° for 18 hours. The reaction mixture was cooled to room temperature and poured onto 2M hydrochloric acid and ethyl acetate. The organic layer was separated, washed with saturated brine, dried ($MgSO_4$) and the solvent was evaporated to give the sub-title compound (8g).
$M^+$265; nmr ($CDCl_3$) δ3.89(3H,s), 3.84(2H,s) and 1.49(9H,s)

b) 3-((3-(Acetylthio)-2(S)-methyl-1-oxopropyl)(carboxymethyl)amino)benzoic acid 1-methyl ester

i) (R)-3-(Acetylthio)-2-methylpropanoyl chloride (3g, 17mmoles), the product of step a) (4g, 15mmoles) and potassium carbonate (4g, 30mmoles) in dichloromethane (50ml) under nitrogen were heated at reflux for 7 hours. The cooled reaction mixture was poured onto dilute aqueous sodium bicarbonate solution and dichloromethane. The organic layer was separated, washed with dilute hydrochloric acid and brine, dried ($MgSO_4$) and the solvent was evaporated.

ii) The residue from i) was dissolved in dichloromethane (15ml) and trifluoroacetic acid (6ml) was added. After stirring for 18 hours, the reaction mixture was poured onto brine/dichloromethane. The organic layer was separated and extracted with aqueous saturated sodium bicarbonate solution. The aqueous extract was acidified with hydrochloric acid and extracted with ethyl acetate. The organic extract was dried ($MgSO_4$) and the solvent was evaporated. Chromatography on silica eluting with dichloromethane and ethyl

acetate/acetic acid gave the sub-title compound (3.3g).
M + 1$^+$ 354; nmr($CDCl_3$) δ3.94(3H,s), 2.30(3H,s) and 1.10(3H,d)

c) 3-((Carboxymethyl)(3-mercapto-2(S)-methyl-1-oxopropyl)amino)benzoic acid 1-methyl ester

Prepared by the method of Example 1(d) and obtained as the free acid mp 114-114.5°.

Example 4

N-(carboxymethyl)-N-(3-mercapto-2(S)-methyl-1-oxopropyl)-β-alanine 2-(dimethylamino)-2-oxoethyl ester dicyclohexylamine salt

a) N-(3-(Acetylthio)-2(S)-methyl-1-oxopropyl)-N-(2-(1,1-dimethylethoxy)-2-oxoethyl)-β-alanine 1-(2-(dimethylamino)-2-oxoethyl) ester

Prepared by the method of Example 1(b).
M + 1$^+$433; nmr δ($CDCl_3$) 2 rotomers 4.74 + 4.71(2xs,2H), 2.33 + 2.31(2xs,3H) and 1.46 + 1.45(2xs,9H).

b) N-(3-(Acetylthio)-2(S)-methyl-1-oxopropyl)-N-(carboxymethyl)-β-alanine 2-(dimethylamino)-2-oxoethyl ester

Prepared by the method of Example 1(c).
M + 1$^+$377; nmr δ($CDCl_3$) 2 rotomers 4.75(s,2H) 2.34 + 2.32(2xs,3H) and 1.23 + 1.17(2xd,3H)

c) N-(Carboxymethyl)-N-(3-mercapto-2(S)-methyl-1-oxopropyl)-β-alanine 1-(2-(dimethylamino)-2-oxoethyl) ester dicyclohexylamine salt

2-Aminoethanethiol (0.6g, 7.8mmole) was added to a stirred solution of the product of step (b) (1.33g, 3.53mmoles) in acetonitrile (20ml) under nitrogen. After 90 minutes the solvent was evaporated and the residue dissolved in ethyl acetate/1% hydrochloric acid. The aqueous phase was separated and re-extracted twice with ethyl acetate. The combined organic extract was dried ($MgSO_4$) and the solvent was evaporated. Chromatography on silica eluting with ethyl acetate/methanol/acetic acid mixtures followed by salt formation with dicyclohexylamine and trituration with diethyl ether gave the title salt (0.3g) mp 102-4°.

Example 5

N-(5-(Benzoylamino)-1,4-dioxo-6-phenylhexyl)-N-(carboxymethyl)-β-alanine 1-(2-(dimethylamino)-2-oxoethyl) ester dicyclohexylamine salt

a) N-((Phenylmethoxy)carbonyl)-β-alanine 2-(dimethylamino)-2-oxoethyl ester

2-Chloro-N,N-dimethylacetamide (1.8g, 15mmoles) was added to a stirred mixture of N-((phenylmethoxy)carbonyl)-β-alanine (3.3g, 15mmoles), triethylamine (1.5g, 15mmoles) and sodium iodide (0.225g) in ethyl acetate (60ml). After heating at reflux for 3 hours, the reaction mixture was cooled and water was added. The organic layer was separated, washed with water and saturated sodium bicarbonate, dried ($Na_2SO_4$) and the solvent was evaporated. Chromatography on silica eluting with ethyl acetate gave the sub-title compound (3.8g).
M$^+$ 308; nmr δ($CDCl_3$) 5.11(s,2H), 4.76(s,2H) and 2.97(s,6H)

b) N-(2-(1,1-Dimethylethoxy)-2-oxoethyl)-β-alanine 2-(dimethylamino)-2-oxoethyl ester

The product of step (a) (7.5g, 24.4mmoles) was hydrogenated at 1 atmosphere of hydrogen over 10% palladium on charcoal (0.5g) in acetic acid (100ml) for 16 hours. The solution was filtered and the solvent was evaporated, excess acetic acid being removed by azeotroping with toluene. The residue in acetonitrile (50ml) was treated with triethylamine (9.7g, 96mmoles) and 1,1-dimethylethoxy bromoacetate (4.75g, 24mmoles). Reaction and work up as in Example 1(a) gave the sub-title compound (3.2g)
M-tBu$^+$, 231; nmr δ($CDCl_3$) 4.74(s,2H), 3.32(s,2H), and 1.46(s,9H)

c) N-(5-(Benzoylamino)-1,4-dioxo-6-phenylhexyl)-N-(carboxymethyl)-β-alanine 1-(2-(dimethylamino)-2-oxoethyl) ester dicyclohexylamine salt

Prepared by the methods of Example F(a) and 1(c) mp 177-9°.

Example 6

N-(5-(Benzoylamino)-1,4-dioxo-6-phenylhexyl)-N-(2-oxo-2-(phenylmethoxy)ethyl)glycine dicyclohexylamine salt

a) N-(2-Oxo-2-(phenylmethoxy)ethyl)glycine 1,1-dimethylethyl ester

Prepared by the method of Example 1(a) using sodium iodide as a catalyst and a reaction time of 4.5 days.
M+1$^+$280; nmr δ($CDCl_3$) 7.32(s,5H), 5.17(s,2H) and 1.46(s,9H)

b) N-(5-(Benzoylamino)-1,4-dioxo-6-phenylhexyl)-N-(2-oxo-2-(phenylmethoxy)ethyl)glycine dicyclohexylamine salt

Prepared by the methods of Example F (a) and 1(c) mp 143-4°.

Example 7

N-(5-(Benzoylamino)-1,4-dioxo-6-phenylhexyl)-N-(2-oxo-2-phenoxyethyl)glycine dicyclohexylamine salt

a) N-(5-(Benzoylamino)-1,4-dioxo-6-phenylhexyl)-N-(2-oxo-2-phenoxyethyl)glycine phenylmethyl ester

Prepared by the method of Example E(a).
M+1$^+$ 607; nmr δ($CDCl_3$) 2 rotomers 5.22+5.18(2xs,2H), 3.34(q,14) and 3.14(q,1H)

b) N-(5-(Benzoylamino)-1,4-dioxo-6-phenylhexyl)-N-(2-oxo-2-phenoxyethyl)glycine dicyclohexylamine salt

The product of step (a) (0.51g, .85mmoles) was hydrogenated at 1 atmosphere in dioxan (50ml) over 10% palladium on carbon (0.05g) for 24 hours. The catalyst was filtered off and the solvent was evaporated. Chromatography on silica eluting with dichloromethane/ethylacetate/acetic acid mixtures followed by salt formation with dicyclohexylamine gave the sub-title compound (0.13g) ex. ether. mp 144.5-145°.

Example 8

S-2-((2-((Carboxymethyl)(3-methoxy-3-oxopropyl)amino)-1(S)-methyl-2-oxoethyl)amino)benzenebutanoic acid trifluoroacetate salt

a) S-2-(4(S)-Methyl-2,5-dioxo-3-oxazolidinyl)benzenebutanoic acid phenylmethyl ester

Bis(trichloromethyl) carbonate (0.17g, 0.6mmoles) was added to a stirred mixture of S-2-((1(S)-carboxyethyl)amino)benzenebutanoic acid phenylmethyl ester (0.5g, 1.47mmoles) in dichloromethane (20ml). The reaction mixture was heated at reflux for 19 hours and the solvent was evaporated to give the sub-title compound.
nmrδ($CDCl_3$) 5.19(s,2H), and 1.45(d,3H).

b) N-(2-oxo-2-(phenylmethoxy)ethyl)-β-alanine methyl ester

Prepared by the method of Example 1(a).
$M-31^+$ 220; nmrδ($CDCl_3$) 5.17(s,2H) and 3.69(s,3H).

c) S-2-((2-((3-Methoxy-3-oxopropyl)(2-oxo-2-(phenylmethoxy)ethyl)amino)-1(S)-methyl-2-oxoethyl)amino)-benzenebutanoic acid phenylmethyl ester trifluoroacetate salt

The product of step (a) (0.58g, 1.6mmoles) and the product of step (b) (0.5g, 2mmoles) was stirred at room temperature for 2.5 days in dichloromethane (10ml). Chromatography on silica eluting with tetrahydrofuran/petroleum ether mixtures followed by salt formation with trifluoroacetic acid gave the sub-title compound (0.59g).
$M+1^+$ 575; nmrδ($CDCl_3$) 2 rotomers 5.1(2xq,2H) and 3.61(s,3H)

d) S-2-((2-((Carboxymethyl)(3-methoxy-3-oxopropyl)amino)-1(S)-methyl-2-oxoethyl)amino)benzenebutanoic acid trifluoroacetate salt

The product of step (c) (0.59g, .86mmoles) was hydrogenated at atmospheric pressure in acetic acid (10ml) using 10% palladium on carbon. After 24 hours, the solvent was evaporated and the residue purified by reverse phase HPLC eluting with water/methanol/trifluoroacetic acid mixtures to give the title compound (0.02g) mp 125-7°.

Example 9

trans-4-(2-Ethoxy-2-oxoethoxy)-1-(3-mercapto-2(S)-methyl-1-oxopropyl)-L-proline dicyclohexylamine salt

a) trans-4-(2-Ethoxy-2-oxoethoxy)-1-((phenylmethoxy) carbonyl)-L-proline phenylmethyl ester

Ethyl diazoacetate (0.5g, 4.2mmoles) in dichloromethane (10ml) was added over 2 hours to a stirred mixture of trans-4-hydroxy-1-((phenylmethoxy)carbonyl)-L-proline phenylmethyl ester (1.4g, 3.95mmoles) and rhodium acetate (catalytic) in dichloromethane (40ml). The solvent was evaporated and the residue chromatographed on silica eluting with ethyl acetate/petroleum ether (60-80°) mixture to give the sub-title compound (0.7g) as an oil.
$M^+$,441; nmr ($CDCl_3$)δ 5.0 and 5.2(2x2H), 1.3(3H)

b) trans-4-(2-Ethoxy-2-oxoethoxy)-L-proline

The product of step (a) (0.7g, 1.6mmole) in acetic acid (30ml) was hydrogenated at 1 atmosphere, over 10% palladium on carbon for 1 day. The catalyst was removed by filtration and the solvent was evaporated

to give the title compound.
$M^{+}$361 (di TMS);
nmr ($D_6$DMSO) δ 4.21(1H), 4.15(2H)

c) trans-1-(3-(Acetylthio)-2(S)-methyl-1-oxopropyl)-4-(2-ethoxy-2-oxoethoxy)-L-proline

(R)-3-(Acetylthio)-2-methylpropanoyl chloride (0.77g, 4.3mmoles) was added to a stirred solution of the product of step (b) (0.4g, 1.84mmoles) and sodium bicarbonate (0.5g) in water (10ml). After stirring overnight, the solution was acidified with 2N hydrochloric acid and extracted (x2) with ethyl acetate. The organic layer was dried ($MgSO_4$) and the solvent was evaporated. The residue was chromatographed on silica eluting with ethyl acetate/acetic acid to give the sub-title compound (0.4g) as an oil.
$M+1^{+}$, 362; nmr ($CDCl_3$) δ 4.2(2H), 4.15(2H), 2.34(3H) and 1.22(3H)

d) trans-4-(2-Ethoxy-2-oxoethoxy)-1-(3-mercapto-2(S)-methyl-1-oxopropyl)-L-proline dicyclohexylamine salt

The product of step (c) (0.4g, 1.1mmoles) in ethanol (5ml) was added dropwise to a solution of lithium ethoxide in ethanol (2ml of 1.6M butyl lithium in 15ml ethanol, 3.2mmoles) under nitrogen. After 1.5 hours, 2N hydrochloric acid (4ml) was added and the solvent was evaporated. The residue was dissolved in ethyl acetate, the organic layer was separated, dried ($MgSO_4$) and the solvent was evaporated. Chromatography on silica eluting with ethyl acetate/acetic acid mixture, followed by salt formation with dicyclohexylamine gave the title compound (0.17) mp 110-112°C

Example 10

trans-1-(N-(1(S)-Carboxy-3-phenylpropyl)-L-alanyl)-4-(2-ethoxy-2-oxoethoxy)-L-proline

a) trans-4-Hydroxy-1-(N-(3-phenyl-1(S)-((phenylmethoxy) carbonyl)propyl)-L-alanyl)-L-proline phenylmethyl ester

Prepared by the method of Example 8(c) and obtained as the free base.
$M+1^{+}$ 545; nmr δ($CDCl_3$) 4.64 (t,1H), 3.37(t,1H) and 1.23 (d,3H)

b) trans-4-(2-Ethoxy-2-oxoethoxy)-1-(N-(3-phenyl-1(S)-((phenylmethoxylcarbonyl)propyl)-L-alanyl)-L-proline phenylmethyl ester

Ethyl diazoacetate (0.5g, 4.3mmoles) in dichloromethane (10ml) was added over 2 hours to a stirred mixture of the product of step (a) (1.4g, 2.57mmoles) and rhodium acetate (catalytic) in dichloromethane (40ml). The reaction mixture was heated at reflux for 1 hour and more ethyl diazoacetate (0.2g) was added. The reaction mixture was chromatographed on silica eluting with ethyl acetate/dichloromethane mixtures and further purified by HPLC to give the title compound (0.3g)
nmr δ($CDCl_3$) 4.37(t,1H), 4.1(q,2H) and 1.17(t,3H).

c) trans-1-(N-(1(S)-Carboxy-3-phenylpropyl)-L-alanyl)-4-(2-ethoxy-2-oxoethoxy)-L-proline

The product of step (b) (0.3g, 0.48mmoles) was hydrogenated at atmospheric pressure in acetic acid (15ml) over 10% palladium on carbon for 24 hours. The catalyst was filtered off and the solvent was evaporated to give the title compound (0.21g) mp115-6°.

Example 11

N-(3-Mercapto-2(S)-methyl-1-oxopropyl)-L-glutamic acid 5-methylester dicyclohexylamine salt

a) N-(3-(Acetylthio)-2(S)-methyl-1-oxopropyl)-L-glutamic acid 5-methyl ester.

(R)-3-(Acetylthio)-2-methylpropanoyl chloride (2.45g, 14 mmoles) was added dropwise to a stirred suspension at 0° of L-glutamic acid 5-methylester (2.0g, 12.4mmoles) and sodium bicarbonate (3.5g, 42mmoles) in water (50ml). After 6 hours the reaction was poured onto 2M hydrochloric acid and ethylacetate. The orgainic layer was separated, washed with brine, dried ($MgSO_4$) and the solvent was evaporated. Chromatography on silica eluting with dichloromethane/ethylacetate/acetic acid followed by trituration with diethylether gave the subtitle compound (1.3g) mp 108-9°

b) N-(3-Mercapto-2(S)-methyl-1-oxopropyl)-L-glutamic acid 5-methylester dicyclohexylamine salt

Potassium hydroxide (0.51g of 85%, 7.8mmoles) in methanol (5ml) was added dropwise to a cooled solution of the product of Example (a) (1.2g, 3.9mmoles) in methanol (50ml) under nitrogen. After 2 hours the solvent was evaporated and the residue was dissolved in 2M hydrochloric acid and ethyl acetate. The organic layer was separated, washed with brine, dried ($mgSO_4$) and the solvent was evaporated to give an oil (0.46g). Dicyclohexylamine (1.76ml of 1M in methanol) was added to a solution of the oil in dichloromethane (20ml). The solvent was evaporated and the resulting mixture was triturated with diethylether to give the title compound (0.57g) mp 146-7°.

Example 12

N-(3-mercapto-2(S)-methyl-1-oxopropyl)-L-aspartic acid 4-methylester dicyclohexylamine salt.

Prepared by the method of Example 11 mp 155.5-156°.

Example 13

(2S-cis)-3-(3-Mercapto-2(S)-methyl-1-oxopropyl)-2,4-thiazolidinedicarboxylic acid 4-methyl ester dicyclohexylamine salt

a) (2S-cis)-3-(3-(Acetylthio)-2(S)-methyl-1-oxopropyl)-2,4-thiazolidinedicarboxylic acid 4-methyl ester

Glyoxylic acid (0.53g, 5.7mmoles) was added to a stirred solution under nitrogen of D-cysteine methyl ester hydrochloride (0.98g, 5.7mmoles) in water, (50ml). After 24 hours, sodium bicarbonate (2.63g, 31mmoles) was added followed by (R)-3-(acetylthio)-2-methylpropanoyl chloride (1.54g, 8.54mmoles). After 2.5 days, the reaction mixture was acidified with hydrochloric acid and saturated with sodium chloride. Extraction with chloroform/ethylacetate was followed by drying ($Na_2SO_4$) and solvent evaporation. Chromatography on silica eluting with ethyl acetate/petroleum ether gave the sub-title compound (0.58g). $M+1^+$ 336; nmr 2 rotamers ($CDCl_3$) 5.67 and 5.43 (2x1H,s), 3.90 (2xs,3H) and 2.36 (2xs,3H).

b) (2S-cis)-3-(3-Mercapto-2(S)-methyl-1-oxopropyl)-2,4-thiazolidinedicarboxylic acid 4-methyl ester dicyclohexylamine salt

Potassium hydroxide (21.8ml of 0.159M solution in methanol, 3.46mmoles) was added to a stirred solution under nitrogen of the product of step (a) (0.58g, 1.73mmoles) in methanol (20ml). After 1.5 hours, the solvent was evaporated and the residue dissolved in dilute hydrochloric acid and ethyl acetate. The organic extract was dried ($Na_2SO_4$) and the solvent was evaporated. Chromatography on silica eluting with dichloromethane/ethylacetate followed by salt formation with dicyclohexylamine and washing with hexane

gave the title compound (0.06g) mp 123-5°

Example 14

trans-4-(2-Ethoxy-2-oxoethoxy)-1-((hydroxy(4-phenylbutyl)phosphinyl)acetyl)-L-proline 1:2 salt with adamantanamine

Carbonyl diimidazole (0.54g, 3.3mmoles) was added to a stirred suspension at 0° of ((hydroxy(4-phenylbutyl) phosphinyl)acetic acid (0.85g, 3.3mmoles) in dichloromethane (20ml). After 1 hour, a solution of trans-4-(2-ethoxy-2-oxoethoxy)-L-proline (0.72g, 3.3mmoles) and triethylamine (1.02g, 10mmoles) in dichloromethane (5ml) was added. After stirring at room temperature for 16 hours, the solvent was evaporated and the residue dissolved in ethyl acetate and phosphate buffer (pH 7). The aqueous layer was separated, acidified to pH 1 with dilute hydrochloric acid and extracted three times with ethyl acetate. These organic extracts were dried ($MgSO_4$) and the solvent was evaporated. One third of this material was converted to the 1:2 salt with adamantanamine (0.36g) and recrystallised from diethyl ether. mp 211-2°.

Example 15

N-(Carboxymethyl)-N-((hydroxy(4-phenylbutyl)phosphinyl)acetyl)-β-alanine-1-methyl ester

a) N-(2-(1,1-Dimethylethoxy)-2-oxoethyl)-N-((hydroxy(4-phenylbutyl)phosphinyl)acetyl)-β-alanine 1-methyl ester

Prepared by the method of Example 14.
$M+1^+$ 456; nmr ($CDCl_3$) 2 rotomers δ 1.47(2xs,9H), 3.65/3.68(2xs,3H)

b) N-(Carboxymethyl)-N-((hydroxy(4-phenylbutyl)phosphinyl)acetyl)-β-alanine 1-methyl ester

Prepared by the method of Example 1(c) mp 145° (decomp.)

Example 16

N-(3-Acetylthio)-2(S)-methyl-1-oxopropyl)-N-(cyclopentyl)-L-glutamic acid 5-methyl ester

a) N-(Cyclopentyl)-L-glutamic acid 5-methyl ester

Cyclopentanone (2.85g, 34 mmoles) was added to a stirred suspension of L-glutamic acid 5-methyl ester (5g, 31 mmoles) and sodium cyanoborohydride (1.36g, 22 mmoles) in methanol (80ml). After 2.5 days, the solution was filtered to give the sub-title compound (3.9g) as a white solid after drying in vacuo.
$M+1^+$ 230; nmr ($D_2O$) δ 3.39(t,1H), 3.46(q,1H), and 3.50(s,3H)

b) N-(3-(Acetylthio)-2(S)-methyl-1-oxopropyl)-N-cyclopentyl)-L-glutamic acid 5-methyl ester

N,O-bistrimethylsilyltrifluoroacetamide (2.25g, 8.75 mmoles) was added to a stirred suspension of the compound of step (a) (1g, 4.37 mmoles) in acetonitrile (30ml). After 30 minutes, (R)-3-(acetylthio)-2-methylpropanoyl chloride (0.8g, 4.44 mmoles) was added. After 7 hours, dilute hydrochloric acid and ethyl acetate were added to the reaction mixture. The organic layer was separated, washed with saturated brine, dried ($MgSO_4$) and the solvent was evaporated. Chromatography on silica eluting with ethyl

acetate/dichloromethane mixtures gave the title compound (1.28g) as a colourless oil.
$M+1^+$ 374; nmr ($CDCl_3$) δ 1.21(d,3H), 2.33(s,3H), 3.68(s,3H)

Example 17

3-((3-(Acetylthio)-2(S)-methyl-1-oxopropyl)(carboxymethyl)amino)pentanedioic acid 1,5-dimethyl ester

a) 3-((2-(1,1-Dimethylethoxy)-2-oxoethyl)amino)pentanedioic acid dimethyl ester

Glycine 1,1-dimethylethyl ester hydrochloride (1g, 6 mmoles), dimethyl 3-oxopentanedioate (1.04g, 6 mmoles) and sodium cyanoborohydride (0.26g, 4 mmoles) in methanol (10ml) were stirred for 1 day. The reaction mixture was acidified with dilute hydrochloric acid and, after 30 minutes, basified with sodium bicarbonate. The reaction mixture was extracted with ethyl acetate. The organic extract was washed with brine, dried ($MgSO_4$), and the solvent was evaporated to give the sub-title compound (1.2g).
$M+1^+$ 290, nmr ($CDCl_3$) δ 1.46(s,9H), 3.69(s,6H)

b) 3-((3-(Acetylthio)-2(S)-methyl-1 oxopropyl)(2-(1,1-dimethylethoxy)-2-oxoethyl)amino)pentanedioic acid dimethyl ester

Prepared by the method of Example 1(b)
$M-OMe^+$ 402

c) 3-((3-(Acetylthio)-2(S)-methyl-1-oxopropyl)(carboxymethyl)amino)pentanedioic acid 1,5-dimethyl ester

Prepared by the method of Example 1(c)
$M+1^+$378 nmr($CDCl_3$) 2 rotomers 2.32/2.34 (2xs,3H), 3.67/3.68/3.69 (3xs,6H).

Example 18

(S-(2α,4α,5α))-1-(3-(Acetylthio)-2(S)-methyl-1-oxopropy)-1-5-phenyl-2,4-pyrrolidinedicarboxylic acid 4-methyl ester

a) (2α,4α,5α)-5-phenyl-2,4-pyrrolidinedicarboxylic acid 4-methyl ester

Prepared by the method of Example 8(d) using (2α,4α,5α)-5-phenyl-2,4-pyrrolidinedicarboxlyic acid 2-(phenylmethyl) 4-methyl ester prepared as in Barr et Al. Tetrahedron 44(2) 557 (1988).
$M^+$ (TMS derivative) 321; nmr ($CDCl_3 + D_6DMSO$) δ 3.2(s,3H), 4.0(t,1H), 4.65(d,1H)

b) (S-(2α,4α,5α)-1-(3-Acetylthio)-2(S)-methyl-1-oxopropyl)-5-phenyl-2,4-pyrrolidinedicarboxylic acid 4-methyl ester

Prepared by the method of Example 9(c). Diastereomers separated by crystallisation from ethyl acetate.
$M+1^+$ 394; nmr ($CDCl_3$) δ 0.84(d,3H), 2.4(s,3H), 3.4(s,3H).

Example 19

The following compounds were prepared according to the method of Example 1 (c):
a) 2-((3-(Acetylthio)-2(S)-methyl-1-oxopropyl)(carboxymethyl)amino)benzoic acid 1-methyl ester

M+1$^{+}$354; nmr δ($CDCl_3$) 2 rotomers 3.95+3.90(2xs,3H) and 2.29+2.25(2xs,3H).

b) 4-((3-(Acetylthio)-2(S)-methyl-1-oxopropyl)(carboxymethyl)amino)benzeneacetic acid 1-methyl ester

M+1$^{+}$ 368; nmr δ($CDCl_3$) 3.73(s,3H), 3.66(s,2H) and 2.30(s,3H)

c) 4-((3-(Acetylthio)-2(S)-methyl-1-oxopropyl)(carboxymethyl)amino)benzenepropanoic acid 1-methyl ester

M+1$^{+}$382; nmr δ($CDCl_3$) 3.69(s,3H), and 2.29(s,3H).

d) N-(3-(Acetylthio)-2(S)-methyl-1-oxopropyl)-N-(carboxymethyl)-D-alanine 1-methyl ester

M+1$^{+}$306; nmr δ($CDCl_3$) 2 rotomers 3.75(s,3H), 2.35(s,3H) a8nd 1.22+1.20(2xd,3H)

e) N-(3-(Acetylthio)-2(S)-methyl-1-oxopropyl)-N-(carboxymethyl)-L-alanine 1-methyl ester

M$^{+}$ for TMS derivative 377; nmr δ($CDCl_3$) 2 rotomers 3.77+3.74(2xs,3H), 2.32(s,3H), 1.23+1.21(2xd,3H)

f) 2-((3-(Acetylthio)-2(S)-methyl-1-oxopropyl)(carboxymethyl)amino)-2-methylpropanoic acid 1-methyl ester

M$^{+}$ for TMS derivative 391; nmr δ($CDCl_3$) 3.79(s,3H), 2.33(s,3H) and 1.65(d,3H)

g) 4-((3-(Acetylthio)-2(S)-methyl-1-oxopropyl) (carboxymethyl)amino)butanoic acid 1-methyl ester

M$^{+}$319; nmr δ($CDCl_3$) 3.69(s,3H), 2.32(s,3H), 1.20(d,3H)

h) N-(3-(Acetylthio)-2(S)-methyl-1-oxopropyl)-N-(carboxymethyl)-β-alanine 1-phenyl ester

M+1$^{+}$368; nmr δ($CDCl_3$)

i) N-(5-(Benzoylamino)-1,4-dioxo-6-phenylhexyl)-N-(carboxymethyl)-β-alanine 1-methyl ester dicyclohexylamine salt

mp 153-5°.

j) (R)-2-((3-(Acetylthio)-2(S)-methyl-1-oxopropyl)carboxymethyl)amino)butanoic acid 1-methyl ester

M$^{+}$ for TMS derivative 391; nmr ($CDCl_3$) 2 rotomers 2.32/2.34(2xs,3H), 3.75/3.78(2xs,3H).

k) N-(3-(Acetylthio)-2(S)-methyl-1-oxopropyl)-N-(carboxymethyl)-D-leucine 1-methyl ester

M+1$^{+}$ 348, nmr ($CDCl_3$) 2 rotomers δ 2.31/2.34(2xs,3H), 3.74/3.75(2xs,3H).

l) N-(3-(Acetylthio)-2(S)-methyl-1-oxopropyl)-N-(carboxymethyl)-D-valine 1-methyl ester

M+1$^{+}$ 334; nmr ($CDCl_3$) 2 rotomers δ 2.30/2.31 (2xs,3H), 3.69/3.70(2xs,3H).

m) N-(3-(Acetylthio)-2(S)-methyl-1-oxopropyl)-N-(carboxymethyl)-D-glutamic acid 1,5-dimethyl ester

M+1$^{+}$ 378; nmr($CDCl_3$) 2 rotomers 2.33/2.34(2xs,3H), 3.68/3.69(2xs,3H), 3.76/3.77(2xs,3H).

n) N-(3-(Acetylthio)-2(S)-methyl-1-oxopropyl)-N-(2-methoxy-2-oxoethyl)-L-alanine

M+1$^{+}$ 306; nmr ($CDCl_3$) 2 rotomers δ 2.32/2.36(2xs,3H), 3.75/3.79(2xs,3H).

o) N-(3-(Acetylthio)-2(S)-methyl-1-oxopropyl)-N-(carboxymethyl)-D-alanine 2-methylpropyl ester

M+1$^{+}$ 348; nmr ($CDCl_3$) 2 rotomers δ 0.92/0.94(2xd,6H), 2.349/2.350(2xs,3H).

p) 5-((3-(Acetylthio)-2(S)-methyl-1-oxopropyl)(carboxymethyl)amino)pentanoic acid 1-methyl ester

M+1$^{+}$ 334; nmr ($CDCl_3$) 2 rotomers δ 2.32/2.33(2xs,3H), 3.67/3.68(2xs,3H)

q) N-(3-(Acetylthio)-2(S)-methyl-1-oxopropyl)-N-(carboxymethyl)-D-norvaline 1-methyl ester

M+1$^{+}$ 334; nmr ($CDCl_3$) 2 rotomers 2.33/2.36(2xs,3H), 3.75/3.77(2xs,3H)

r) N-(3-(Acetylthio)-2(S)-methyl-1-oxopropyl)-N-(carboxymethyl)-D-phenylalanine 1-methyl ester

M+1$^{+}$ 382; nmr ($CDCl_3$) 2 rotomers δ 0.91/1.20(2xd,3H), 2.31(2xs,3H)

s) 1-(3-(Acetylthio)-2(S)-methyl-1-oxopropyl)-1,2-hydrazinediacetic acid 2-methyl ester

M+1$^{+}$ 307; nmr ($CDCl_3$) δ 2.33(s,3H), 3.76(s,3H)

t) N-(3-(Acetylthio)-2(S)-methyl-1-oxopropyl)-N-(2- methoxy-2-oxoethoxy)glycine

M-15 (diTMS derivative) 436; nmr ($CDCl_3$) δ 2.34(s,3H), 3.78(s,3H).

u) 4-((3-(Acetylthio)-2(S)-methyl-1-oxopropyl)(carboxymethyl)amino)benzoic acid 1-methyl ester

M+1$^{+}$ 354; nmr ($CDCl_3$) 2 rotomers δ 2.30/2.35(2xs,3H), 3.95(s,3H).

v) N-(3-(Acetylthio)-2(S)-methyl-1-oxopropyl)-N-(4-(2-methoxy-2-oxoethoxy)phenyl)glycine

mp 120-120.5°.

w) (2R-cis)-1-(3-(Acetylthio)-2(S)-methyl-1-oxopropyl)-5-carboxy-2-pyrrolidineacetic acid 2-methyl ester

M+1$^{+}$332; nmr δ($CDCl_3$) 2 rotomers 1.16/1.25 (2xd,3H), 3.70 (2xs,3H).

x) N-(3-(Acetylthio)-2(S)-methyl-1-oxopropyl)-N-(carboxymethyl)-β-alanine 2-methylpropyl ester

M+1$^{+}$ 348; nmr ($CDCl_3$) 2 rotomersδ 0.92/0.93(2xd,6H), 2.32/2.34(2xs,3H)

y) N-(3-(Acetylthio)-2(S)-methyl-1-oxopropyl)-N-(carboxymethyl)-β-alanine ethyl ester

M+1$^{+}$ 320; nmr ($CDCl_3$) 2 rotomers δ 2.32/2.34(2xs,3H)

z) 4-((3-(Acetylthio)-2(S)-methyl-1-oxopropyl)(carboxymethyl)amino)butanoic acid 1-(2-methylpropyl)-ester

M+1$^{+}$362; nmr δ($CDCl_3$) 2 rotomers 1.03(d,6H), 1.29/1.33 (2xd,3H), 2.44/2.49 (2xs,3H)

aa) (R)-3-((3-(Acetylthio)-2(S)-methyl-1-oxopropyl)(carboxymethyl)amino)butanoic acid 1-methyl ester

M+1⁺320; nmr δ (s,3H), 3.66 (s,3H)

ab) N-(3-(Acetylthio)-2(S)-methyl-1-oxopropyl)-N-(carboxymethyl)-L-glutamic acid 1,5-dimethyl ester
nmr δ ($CDCl_3$) 1.20/1.23 (2xd,3H), 3.68-3.77 (4xs,6H).

ac) N-(3-(Acetylthio)-2(S)-methyl-1-oxopropyl)-N-(2-methoxy-2-oxoethyl)glycine
$M^+$ (TMS derivative) 363; nmr ($CDCl_3$) 2 rotomers δ 2.335/2.337(2xs,3H), 3.77/3.79(2xs,3H).

Example 20

The following compounds were prepared according to the method of Example 1 (d):

a) 2-((Carboxymethyl)(3-mercapto-2(S)-methyl-1-oxopropyl)amino)benzoic acid 1-methyl ester dicyclohexylamine salt
mp 143-4°.

b) 4-((Carboxymethyl)(3-mercapto-2(S)-methyl-1-oxopropyl)amino)benzeneacetic acid 1-methyl ester dicyclohexylamine salt
mp 157-8°.

c) 4-((Carboxymethyl)(3-mercapto-2(S)-methyl-1-oxopropyl)amino)benzenepropanoic acid 1-methyl ester dicyclohexylamine salt
mp 125.5°-126°.

d) N-(Carboxymethyl)-N-(3-mercapto-2(S)-methyl-1-oxopropyl)-D-alanine 1-methyl ester dicyclohexylamine salt
mp 123-5°.
$(\alpha)^D{}_{23}$ = +13.62° c=0.22(MeOH)

e) N-(Carboxymethyl)-N-(3-mercapto-2(S)-methyl-1-oxopropyl)-L-alanine 1-methyl ester dicyclohexylamine salt
mp 137-9°
$[\alpha]^D{}_{23}$ = 40°-87° C=0.26($CHCl_3$)

f) 2-((Carboxymethyl)(3-mercapto-2(S)-methyl-1-oxopropyl)amino)-2-methylpropanoic acid 1-methyl ester dicyclohexylamine salt
mp 120.5°-121°.

g) 4-((Carboxymethyl)(3-mercapto-2(S)-methyl-1-oxopropyl)amino)butanoic acid 1-methyl ester 1-adamantanamine salt
mp 133.5-134.5° $[\alpha]^D$ = 15.34° (C=0.3,MeOH)

h) N-(Cyclopentyl)-N-(3-mercapto)-2(S)-methyl--1-oxopropyl)-L-glutamic acid 5-methyl ester
mp 91-2°.

i) N-(Carboxymethyl)-N-(3-mercapto-2(S)-methyl-1-oxopropyl)-D-leucine 1-methyl ester adamantanamine salt
mp 197-8°.

j) 3-((Carboxymethyl)(3-mercapto-2(S)-methyl-1-oxopropyl)amino)pentanedioic acid 1,5-dimethyl ester adamantanamine salt
mp 144.5-5°.

k) (S-(2α,4α,5α))-1-(3-mercapto-2(S)-methyl-1-oxopropyl)-5-phenyl-2,4-pyrrolidinedicarboxylic acid 4-methyl ester
mp 115-6°.

l) N-(Carboxymethyl)-N-(3-mercapto-2(S)-methyl-1-oxopropyl)-D-valine 1-methyl ester dicyclohexylamine salt
mp 140-1°.

m) N-(Carboxymethyl)-N-(3-mercapto-2(S)-methyl-1-oxopropyl)-D-glutamic acid 1,5-dimethyl ester adamantanamine salt
mp 166-7°.

n) (2α,4α,5α)-1-(3-mercapto-1-oxopropyl)-5-phenyl-2,4-pyrrolidinedicarboxylic acid 4-methyl ester
mp 202-3°.

o) N-(3-(Mercapto-2(S)-methyl-1-oxopropyl)-N-(2-methoxy-2-oxoethyl)-L-alanine dicyclohexylamine salt
mp 99-101°.

p) N-(Carboxymethyl)-N-(3-mercapto-2(S)-methyl-1-oxopropyl)-D-alanine 2-methylpropyl ester adamantanamine salt
Using 2-methylpropanol as solvent mp 185-7°.

q) 5-((Carboxymethyl)(3-mercapto-2(S)-methyl-1-oxopropyl)amino)pentanoic acid 1-methyl ester adamantanamine salt
mp 125-6°.

r) N-(Carboxymethyl)-N-(3-mercapto-2(S)-methyl-1-oxopropyl)-D-norvaline 1-methyl ester dicyclohexylamine salt
mp 91.5-2°.

s) N-(Carboxymethyl)-N-(3-mercapto-2(S)-methyl-1-oxopropyl)-D-phenylalanine 1-methyl ester adamantanamine salt
mp 211-3°.

t) N-(Carboxymethyl)-N-(3-mercapto-2(S)-methyl-1-oxopropyl)-β-alanine 1-ethyl ester adamantanamine salt
Using ethanol as solvent mp 141-2°.

u) N-(Carboxymethyl)-N-(3-mercapto-2(S)-methyl-1-oxopropyl)-L-glutamic acid 1,5-dimethyl ester adamantanamine salt
mp 168-9°.

v) N-Carboxymethyl)-N-(3-mercapto-2(S)-methyl-1-oxopropyl)-β-alanine 1-(2-cyclohexylethyl) ester adamantanamine salt
mp 118-20°

w) N-(Carboxymethyl)-N-(3-mercapto-2(S)-methyl-1-oxopropyl)-β-alanine 1-cyclohexyl ester adamantanamine salt
mp 145-6°

x) (R)-3-((Carboxymethyl)(3-mercapto-2(S)-methyl-1-oxopropyl)amino)butanoic acid 1-methyl ester adamantanamine salt
mp 182-3°

y) 4-((Carboxymethyl)(3-mercapto-2(S)-methyl-1-oxopropyl)amino)butanoic acid 1-(2-methylpropyl) ester adamantanamine salt
Using 2-methylpropanol as solvent mp 127-8°

z) N-(Carboxymethyl)-N-(3-mercapto-2(S)-methyl-1-oxopropyl)-β-alanine 1-(2-methylpropyl) ester adamantanamine salt
Using 2-methylpropanol as solvent
$M+1^+$ 457; nmr δ ($D_6$DMSO) 0.88 (d,6H)

Example 21

The following compounds were prepared according to the method of Example 4 (c):

a) N-(Carboxymethyl)-N-(3-mercapto-2(S)-methyl-1-oxopropyl)-β-alanine 1-phenyl ester dicyclohexylamine salt
mp 119-20°.

b) (R)-2-((Carboxymethyl)(3-mercapto-2(S)-methyl-1-oxopropyl)amino)butanoic acid 1-methyl ester dicyclohexylamine salt
mp 131.5-132.5

Example 22

The following compounds were prepared according to the method of Example 9 (c):

a) (2α,4α,5α)-1-(3-(Acetylthio)-1-oxopropyl)-5-phenyl-2,4-pyrrolidinedicarboxylic acid 4-methyl ester
$M+1^+$ 380; nmr ($CDCl_3$) δ 2.21(s,3H), 3.40(s,3H), 5.25(d,1H).

b) N-(3-(Acetylthio)-2(S)-methyl-1-oxopropyl)-L-aspartic acid 4-methyl ester
$M^+$ (TMS derivative) 363; nmr ($CDCl_3$) δ(s,3H), 3.74(s,3H).

Example 23

N-(3-(Acetylthio)-2(S)-methyl-1-oxopropyl)-N-(carboxymethyl)-β-alanine 1-cyclohexyl ester

a) $\beta$-Alanine 1-cyclohexyl ester hydrochloride

Chlorotrimethylsilane (10ml) was added to a stirred suspension of $\beta$-alanine hydrochloride (1g, 11.2 mmoles) in cyclohexanol (12ml). After stirring for 24 hours, the excess reagents were removed in vacuo and the residue triturated with diethyl ether/petroleum ether to give the sub-title compound (2g).
$M^+$ 171; nmr($CDCl_3$) $\delta$ 2.9(t,2H), 3.39(t,2H), 4.80(m,1H)

b) N-(2-(1,1-Dimethylethoxy)-2-oxoethyl)-$\beta$-alanine cyclohexyl ester

Prepared by the method of Example 1(a)
$M+Rb^+$ 370; nmr($CDCl_3$) $\delta$ 1.50(s,9H), 2.52(t,2H), 2.90(t,2H).

c) N-(3-(Acetylthio)-2(S)-methyl-1-oxopropyl)-N-(2-(1,1-dimethylethoxy)-2-oxoethyl)-$\beta$-alanine cyclohexyl ester

Prepared by the method of Example 1(b)
nmr($CDCl_3$) 2 rotomers 1.50/1.51 (2xs,9H) 2.33/2.35 (2xs,3H).

d) N-(2-(Acetylthio)-2(S)-methyl-1-oxopropyl)-N-(carboxymethyl)-$\beta$-alanine 1-cyclohexyl ester

Prepared by the method of Example 1(c).
$M+1^+$374; nmr $\delta$($CDCl_3$) 2 rotomers 1.19/1.25 (2xd,3H), 2.34/2.35 (2xs,3H).

Example 24

N-(3-(Acetylthio)-2(S)-methyl-1-oxopropyl)-N-(carboxymethyl)-$\beta$-alanine 1-(2-cyclohexylethyl) ester

a) $\beta$-Alanine 2-cyclohexylethyl ester hydrochloride
Prepared by the method of Example 23(a)
nmr ($CDCl_3$) $\delta$ 2.93(t,2H), 4.16(t,2H).
b) N-(2-(1,1-Dimethylethoxy)-2-oxoethyl)-$\beta$-alanine 2-cyclohexylethyl ester
Prepared by the method of Example 1(a)
nmr ($CDCl_3$) $\delta$ 1.45(s,9H), 2.50(t,2H), 2.87(t,2H), 3.30(s,2H).
c) N-(3-(Acetylthio)-2(S)-methyl-1-oxopropyl)-N-(2-(1,1-dimethylethoxy)-2-oxoethyl)-$\beta$-alanine 2-cyclohexylethyl ester
Prepared by the method of Example 1(b)
nmr ($CDCl_3$) 2 rotomers $\delta$ 1.52/1.54 (2xs,9H), 2.35/2.36 (2xs,3H)
d) N-(3-(Acetylthio)-2(S)-methyl-1-oxopropyl)-N- (carboxymethyl)-$\beta$-alanine 1-(2-cyclohexylethyl) ester
Prepared by the method of Example 1(c)
$M+1^+$402; nmr $\delta$ ($CDCl_3$) 2 rotomers 1.20/1.25 (2xd, 3H), 2.33/2.34 (2xs,3H).

Example 25

(2S-trans)-1-(3-(Acetylthio)-2(S)-methyl-1-oxopropyl)-2,4-pyrrolidinedicarboxylic acid 4-methyl ester

a) trans-2,4-Pyrrolidinedicarboxylic acid 4-methyl ester

Prepared by the method of Example 8(d) using trans-2,4-pyrrolidinedicarboxylic acid 2-phenylmethyl 4-methyl ester prepared by the method of Kravs et al. Tetrahedran 41(17)3527 (1985)
$M+1^+$174; nmr($D_6$DMSO) $\delta$ 2.20(t,2H), 2.20 (t,1H), 3.19(t,1H), 3.64(s,3H)

b) (2S-trans)-1-(3-(Acetylthio)-2(S)-methyl-1-oxopropyl)-2,4-pyrrolidinedicarboxylic acid 4-methyl ester

Prepared by the method of Example 9(c) as a mixture of diastereomers.
$M+1^+$ 318; nmr δ ($CDCl_3$) 1.24(d,3H), 2.35/2.36 (2xs,3H), 3.75 (s,3H).

Example 26

(2R-cis)-3-(3-(Acetylthio)-2(S)-methyl-1-oxopropyl)-2,4-thiazolidinedicarboxylic acid 2-methyl ester

a) (2R-cis)-2,4-Thiazolidinedicarboxylic acid 2-methyl ester

Methyl glyoxylate (1.6g, 19mmoles) in methanol (10ml) was added to a stirred mixture of L-cysteine hydrochloride hydrate (2.4g, 20mmoles) and sodium acetate (2.5g) in water (20ml). After stirring for 16 hours, the solid was filtered, washed with diethyl ether and dried in vacuo to give the sub-title compound (1.6g).
$M+1^+$ 192; nmr δ ($D_6$DMSO) 3.73(s,3H), 5.02(s,1H)

b) (2R-cis)-3-(3-(Acetylthio)-2(S)-methyl-1-oxopropyl)-2,4-thiazolidinedicarboxylic acid 2-methyl ester

Prepared by the method of Example 9(c)
nmr δ($CDCl_3$) 2 rotomers 2.36/2.37 (2xs,3H), 3.89/3.94 (2xs,3H), 5.78/5.88 (2xs,1H).

INTERMEDIATES

Example A

2-((2-(1,1-Dimethylethoxy)-2-oxoethyl)amino)benzoic acid methyl ester

Prepared by the method of Example 3(a).
$M^+$ 265; nmr δ($CDCl_3$) 3.85(S,3H) and 1.48(S,9H)

Example B

2-((3-(Acetylthio)-2(S)-methyl-1-oxopropyl)(2-(1,1-dimethylethoxy)-2-oxoethyl)amino)benzoic acid methyl ester

Prepared by the method of Example 3b(i) and purified by chromatography.
$M^+$ 409; nmr δ($CDCl_3$) 2 rotomers 3.93/3.87 (2xs,3H), 2.28/2.25 (2xs,3H), 1.49/1.48 (2xs,9H).

Example C

4-((2-(1,1-Dimethylethoxy)-2-oxoethyl)amino)benzenepropanoic acid methyl ester

Prepared by the method of Example 3(a), reaction being run at 25° for 24 hours.
$M^+$ 293; nmr δ($CDCl_3$) 3.77(s,2H), 3.66(s,3H) and 1.48(s,9H)

Example D

4-((2-(1,1-Dimethylethoxy)-2-oxoethyl)amino)butanoic acid methyl ester

Prepared by the method of Example 3(a) at room temperature.
M+1$^+$232; nmr δ($CDCl_3$) 3.67(s,3H) and 1.46(s,9H)

Example E

β-Alanine phenyl ester trifluoroacetate salt

a) N-((1,1-Dimethylethoxy)carbonyl)-β-alanine phenyl ester

(1-Benzotriazolyloxy)(tris(dimethylamino))phosphonium hexafluorophosphate (4.68g, 10.6mmoles) was added to a solution of N-((1,1-dimethylethoxy)carbonyl)-β-alanine (2.0g, 10.6mmoles), phenol (1.0g, 10.6mmoles) and triethylamine (2.16g, 21.4mmoles) in dichloromethane (160ml). After 16.5 hours at room temperature, brine and ethyl acetate were added. The organic layer was separated, washed with 2N hydrochloric acid, saturated sodium bicarbonate and brine dried ($MgSO_4$) and the solvent was evaporated to give the title compound as a white solid.
M-56$^+$ 209; nmr δ($CDCl_3$) 3.50(q,2H), 2.80(t,2H) and 1.45(s,9H).

b) β-Alanine phenyl ester trifluoroacetate salt

Prepared by the method of Example 1(c).
M+1$^+$ 166; mnr δ($CDCl_3$) 7.37(t,2H), 7.24(t,1H), 7.05(d,2H) and 2.88(t,2H).

Example F

N-(5-(Benzoylamino)-1,4-dioxo-6-phenylhexyl)-N-(2-(1,1-dimethylethoxy)-2-oxoethyl)-β-alanine methyl ester

Dicyclohexylcarbodiimide (1.13g, 5.5mmoles) in dichloromethane (10ml) was added to an ice-cooled solution of 5-(benzoylamino)-4-oxo-6-benzenehexanoicacid (1.6g, 5mmoles), 1-hydroxybenzotriazole (0.68g, 5mmoles) and N-(2-(1,1-dimethylethoxy)-2-oxoethyl)-β-alanine methyl ester (1.08g, 5mmoles) in dichloromethane (40ml). After stirring for 16 hours, the solution was filtered. The filtrate was washed with 2N hydrochloric acid, water and saturated sodium bicarbonate, dried ($Na_2SO_4$) and the solvent was evaporated. Chromatography on silica eluting with ethyl acetate/petroleum ether mixture gave the sub-title compound as an oil (1.85g).
M+1$^+$ 525; nmr δ($CDCl_3$) 2 rotomers 3.70 and 3.67(2xs,3H) and 1.48 and 1.44(2xs,9H)

Example G

1-(3-(Acetylthio)-2(S)-methyl-1-oxopropyl)-1-((1,1 -dimethylethoxy)carbonyl)-1,2-hydrazineacetic acid 2-methyl ester

Prepared by the method of Example 3(a) using the product of Example N(d).
M+1$^+$ 463

Example H

(((3-(Acetylthio)-2(S)-methyl-1-oxopropyl)amino)oxy)acetic acid methyl ester

Prepared by the method of Example 3(b)(i) at room temperature.
$M+1^+$ 250; nmr ($CDCl_3$) δ 2.34(s,3H), 3.31(s,3H)

Example I

4-((3-(Acetylthio)-2(S)-methyl-1-oxopropyl)(2-(1,1-dimethylethoxy)-2-oxoethyl)amino)benzoic acid methyl ester

a) 4-((2-(1,1-Dimethylethoxy)-2-oxoethyl)amino)benzoic acid methyl ester
Prepared by the method of Example 3(a).
$M^+$ 265; nmr ($CDCl_3$) δ 1.50(s,9H), 3.85(s,2H)

b) 4-((3-(Acetylthio)-2(S)-methyl-1-oxopropyl)(2-(1,1-dimethylethoxy)-2-oxoethyl)amino)benzoic acid methyl ester
Prepared by the method of Example 3(b)(i).
$M+1^+$ 410; nmr ($CDCl_3$) 2 rotomers δ 1.47(s,9H), 2.29/2.35(2xs,3H), 3.94(s,3H).

Example J

(2R-cis)-5-((1,1-Dimethylethoxy)carbonyl)-2-pyrrolidineacetic acid methyl ester

(2R-cis)-5-((1,1-Dimethylethoxy)carbonyl)-1-(phenylmethyl)-2-pyrrolidineacetic acid methyl ester (2.83g, 8.4mmoles) was hydrogenated at 3 atmospheres over 10% palladium/charcoal (0.3g, 50% moisture) in acetic acid (35ml) for 21 hours. The solution was filtered and the solvent was evaporated. The residue was dissolved in ethyl acetate and saturated sodium bicarbonate. The organic layer was separated, washed with brine, dried ($MgSO_4$) and the solvent was evaporated to give the sub-title compound (1.47g).
$M+1^+$ 244; nmr ($CDCl_3$) δ 1.47(s,9H) 3.7(s,3H).

Example K

4-((Carboxymethyl)(3-mercapto-2(S)-methyl-1-oxopropyl)amino)benzeneacetic acid 1-methyl ester dicyclohexylamine salt

Prepared by the method of Example 3(a), reaction being run at 25° for 24 hours.
$M^+$293; nmr δ($CDCl_3$) 3.77(s,2H), 3.66(s,3H) and 1.48(s,9H)

Example L

The following intermediates were prepared according to the method of Example 1(a):

a) 4-((2-(1,1-Dimethylethoxy)-2-oxoethyl)amino)benzeneacetic acid methyl ester
$M^+$279; nmr δ($CDCl_3$) 3.78(s,2H), 3.67(s,3H) and 1.49(s,9H)

b) N-(2-(1,1-Dimethylethoxy)-2-oxoethyl)-D-alanine methyl ester
$M+1^+$ 218; nmr δ($CDCl_3$) 3.73(s,3H), 1.46(s,9H) and 1.34(d,3H)

c) N-(2-(1,1-Dimethylethoxy)-2-oxoethoxy)-L-alanine methyl ester
$M+1^+$218; nmr δ ($CDCl_3$) 3.73(s,3H), 1.46(s,9H) and 1.34(d,3H)

d) 2-((2-(1,1-Dimethylethoxy)-2-oxoethyl)amino)-2-methylpropanoic acid methyl ester
$M+1^+$ 232; nmr δ ($CDCl_3$) 3.68(s,3H) 2.32(s,3H) and 1.48(s,9H)

e) N-(2-(1,1-Dimethylethoxy)-2-oxoethyl)-β-alanine phenyl ester
$M+1^+$280; nmr δ($CDCl_3$) 3.35(s,2H), 1.47(s,9H)

f) N-(2-(1,1-Dimethylethoxy)-2-oxoethyl)-D-leucine methyl ester
$M+1^+$ 260, nmr ($CDCl_3$) δ 1.49(s,9H), 3.72(s,3H).

g) N-(2-(1,1-Dimethylethoxy)-2-oxoethyl)-D-valine methyl ester
$M^+$ 245; nmr ($CDCl_3$) 1.46(s,9H), 3.73(s,3H)
h) N-(2-Methoxy-2-oxoethyl)-L-alanine 1,1-dimethylethyl ester
M-56$^+$; nmr ($CDCl_3$) δ 1,47(s,9H), 3.73(s,3H).
i) N-(2-(1,1-Dimethylethoxy)-2-oxoethyl)-D-alanine 2-methylpropyl ester
M+1$^+$ 260; nmr ($CDCl_3$) δ 0.94(d,6H), 1.46(s,9H).
j) 5-((2-(1,1-Dimethylethoxy)-2-oxoethyl)amino)pentanoic acid methyl ester
$M^+$ 245; nmr ($CDCl_3$) δ 1.47(s,9H), 3.67(s,3H)
k) N-(2-(1,1-Dimethylethoxy)-2-oxoethyl-D-phenylalanine methyl ester
$M^+$ 293; nmr ($CDCl_3$) δ 1.42(s,9H), 3.65(s,3H)
l) N-(2-(1,1-Dimethylethoxy)-2-oxoethyl)-β-alanine 2-methylpropyl ester
M+1$^+$ 260; nmr ($CDCl_3$) 0.93(d,6H), 1.47(s,9H), 3.31(s,2H)
m) N-(2-(1,1-Dimethylethoxy)-2-oxoethyl)-β-alanine ethyl ester
M+1$^+$ 232; nmr δ($CDCl_3$) 1.47(s,9H), 3.31(s,2H)
n) 4-((2-(1,1-Dimethylethoxy)-2-oxoethyl)amino)butanoic acid 2-methylpropyl ester
M-56$^+$ 217; nmr ($CDCl_3$)δ 0.92(d,6H), 1.47(s,9H), 3.28(s,2H)
o) N-(2-(1,1-dimethylethoxy)-2-oxoethyl)glycine methyl ester
M-56$^+$ 147; nmr ($CDCl_3$) δ 1.47(s,9H), 3.74(s,3H)

Example M

The following intermediates were prepared according to the method of Example 1(b):

a) 4-((3-(Acetylthio)-2(S)-methyl-1-oxopropyl)(2-(1,1-dimethylethoxy)-2-oxoethyl)amino)benzeneacetic acid methyl ester
$M^+$ 423; nmr δ($CDCl_3$) 3.73(s,3H), 3.65(s,2H) and 2.29(s,3H).

b) N-(3-(Acetylthio)-2(S)-methyl-1-oxopropyl)-N-(2-(1,1-dimethylethoxy)-2-oxoethyl)-D-alanine methyl ester
M+1$^+$ 362; nmr δ($CDCl_3$) 2 rotomers 3.71(s,3H), 2.34(s,3H) and 1.47(s,9H).

c) N-(3-(Acetylthio)-2(S)-methyl-1-oxopropyl)-N-(2-(1,1-dimethylethoxy)-2-oxoethyl)-L-alanine methyl ester
M+1$^+$ 362; nmr δ ($CDCl_3$) 2 rotomers 3.74+3.69(2xs,3H), 2.32+2.31(2xs,3H) and 1.47+1.44(2xs,9H)

d) 2-((3-(Acetylthio)-2(S)-methyl-1-oxopropyl)(2-(1,1-dimethylethoxy)-2-oxoethyl)amino)-2-methylpropanoic acid methyl ester
M+1$^+$ 376; nmr δ ($CDCl_3$) 3.68(s,3H), 2.32(s,3H), 1.48(s,9H)

e) 4-((3-Acetylthio)-2(S)-methyl-1-oxopropyl)(2-(1,1- dimethylethoxy)-2-oxoethyl)amino)butanoic acid methyl ester
M+1$^+$ 376; nmr δ ($CDCl_3$) 3.70(s,3H), 2.32(s,3H) and 1.45(s,9H)

f) N-(3-(Acetylthio)-2(S)-methyl-1-oxopropyl)-N-(2-(1,1-dimethylethoxy)-2-oxoethyl)-β-alanine phenyl ester
M+1$^+$ 424; nmr δ ($CDCl_3$) 2 rotomers 2.30+2.28(2xs,3H), 1.46+1.45(2xs,9H)

g) (R)-2-((3-(Acetylthio)-2(S)-methyl-1-oxopropyl)(2-(1,1-dimethylethoxy)-2-oxoethyl)amino)butanoic acid methyl ester
M+1$^+$ 376; nmr δ ($CDCl_3$) 2 rotomers 2.31 and 2.33(2xs,3H) and 3.70 and 3.72(2xs,3H)

h) N-(3-(Acetylthio)-2(S)-methyl-1-oxopropyl)-N-(2-(1,1-dimethylethoxy)-2-oxoethyl)-D-leucine methyl ester
M+1$^+$ 404; nmr δ ($CDCl_3$) 2 rotomers 1.44/1.47(2xs,9H), 2.31/2.33(2xs,3H), 3.69/3.72(2xs,3H).

i) N-(3-(Acetylthio)-2(S)-methyl-1-oxopropyl)-N-(2-(1,1-dimethylethoxy)-2-oxoethyl)-D-valine methyl ester
M+1$^+$ 390; nmr δ ($CDCl_3$) 2 rotomers 1.45/1.47(2xs,9H), 2.34/2.35(2xs,3H), 3.69/3.71(2xs,3H).

j) N-(3-(Acetylthio)-2(S)-methyl-1-oxopropyl)-N-(2-(1,1-dimethylethoxy)-2-oxoethyl)-D-glutamic acid dimethyl ester
M+1$^+$ 434; nmr δ ($CDCl_3$) 2 rotomers 2.33/2.34(2xs,3H), 3.6/3.7(4xs,6H).

k) N-(3-Acetylthio)-2(S)-methyl-1-oxopropyl)-N-(2-methoxy-2-oxoethyl)-L-alanine 1,1-dimethylethyl ester
M+1$^+$ 362; nmr δ ($CDCl_3$) 2 rotomers 1.43/1.46(2xs,9H), 2.30/2.32(2xs,3H), 3.71/3.76(s,3H).

l) N-(3-(Acetylthio)-2(S)-methyl-1-oxopropyl)-N-(2-(1,1-dimethylethoxy)-2-oxoethyl)-D-alanine 2-methylpropyl ester

M + 1$^{+}$ 404; nmr δ ($CDCl_3$) 2 rotomers 1.46/1.47(2xs,9H), 2.336/2.345(2xs,3H).

m) 5-((3-(Acetylthio)-2(S)-methyl-1-oxopropyl)(2-(1,1-dimethylethoxy)-2-oxoethyl)amino)pentanoic acid methyl ester

No polyvinylpyridine or dimethylaminopyridine added.

M + 1$^{+}$ 390; nmr δ ($CDCl_3$) 2 rotomers 1.46/1.47(2xs,9H), 2.33/2.34(2xs,3H), 3.66/3.68(2xs,3H)

n) N-(3-(Acetylthio)-2(S)-methyl-1-oxopropyl)-N-(2-(1,1-dimethylethoxy)-2-oxoethyl)-D-norvaline methyl ester

M + 1$^{+}$ 390; nmr δ ($CDCl_3$) 2 rotomers 1.44/1.47(2xs,9H), 2.32/2.33(2xs,3H), 3.68/3.72(2xs,3H).

o) N-(3-(Acetylthio-2(S)-methyl-1-oxopropyl)-N-(2-(1,1-dimethylethoxy)-2-oxoethyl)-D-phenylalanine methyl ester

M + 1$^{+}$ 438; nmr δ ($CDCl_3$) 1.44(s,9H), 2.30(s,3H)

p) N-(3-(Acetylthio)-2(S)-methyl-1-oxopropyl)-N-(4-(2- methoxy-2-oxoethoxy)phenyl)glycine 1,1-dimethylethyl ester

M$^{+}$ 439.

q) (2R-cis)-1-(3-(Acetylthio)-2(S)-methyl-1-oxopropyl)-5-((1,1-dimethylethoxy)carbonyl)-2-pyrrolidineacetic acid methyl ester

M + 1$^{+}$ 388; nmr δ ($CDCl_3$) 2 rotomers 1.46(2xs,9H), 2.31(2,s,3H), 3.7(2xs,3H)

r) N-(3-(Acetylthio)-2(S)-methyl-1-oxopropyl)-N-(2-(1,1-dimethylethoxy)-2-oxoethyl)-β-alanine 2-methylpropyl ester

M + 1$^{+}$ 404; nmr δ ($CDCl_3$) 2 rotomers 0.92/0.93(2xd,6H), 1.456/1.464(2xs,9H)

s) N-(3-(Acetylthio)-2(S)-methyl-1-oxopropyl)-N-(2-(1,1-dimethylethoxy)-2-oxoethyl)-β-alanine ethyl ester

M + 1$^{+}$ 376; nmr δ ($CDCl_3$) 2 rotomers 1.46/1.47(2xs,9H), 2.31/2.34(2xs,3H)

t) N-(3-(Acetylthio)-2(S)-methyl-1-oxopropyl)-N-(2-(1,1-dimethylethoxy)-2-oxoethyl)-L-glutamic acid dimethyl ester

M + 1$^{+}$ 434; nmr δ ($CDCl_3$) 2 rotomers 1.21/1.23 (2xd,3H), 1.46/1.48(2xs,9H), 3.67-3.76(4xs,6H)

u) 4-((3-(Acetylthio)-2(S)-methyl-1-oxopropyl)(2-(1,1-dimethylethoxy)-2-oxoethyl)amino)butanoic acid 2-methylpropyl ester

M + 1$^{+}$ 418; nmr δ ($CDCl_3$) 2 rotomers 0.92/0.93(2xd,6H), 1.46/1.47(2xs,9H), 2.32/2.33(2xs,3H).

v) (R)-3-((3-(Acetylthio)-2(S)-methyl-1-oxopropyl)(2-(1,1-dimethylethoxy)-2-oxoethyl)amino)butanoic acid methyl ester

M + 1$^{+}$ 376; nmr δ ($CDCl_3$) 2 rotomers 1.45/1.46(2xs,9H), 2.32/2.35(2xs,3H), 3.77/3.79(2xs,3H)

w) N-(3-(Acetylthio)-2(S)-methyl-1-oxopropyl)-N-(2-(1,1-dimethylethoxy)-2-oxoethyl)glycine methyl ester

M-56$^{+}$ 291; nmr δ ($CDCl_3$) 2 rotomers 1.46/1.47(2xs,9H), 2.33(s,3H), 3.73/3.77(s,3H)

x) 2-(3-(Acetylthio)-2(S)-methyl-1-oxopropyl) hydrazinecarboxylic acid 1,1-dimethylethyl ester

M + 1$^{+}$ 277; nmr δ ($CDCl_3$) 1.47(s,9H). 2.35(s,3H)

## Example N

The following intermediates were prepared according to the method of Example 3(a):

a) (R)-2-((2-(1,1-Dimethylethoxy)-2-oxoethyl)amino)butanoic acid methyl ester

M + 1$^{+}$ 232; nmr δ ($CDCl_3$) 1.46(s,9H), 3.73(s,3H)

b) N-(2-(1,1-Dimethylethoxy)-2-oxoethyl)-D-glutamic acid dimethyl ester

M + 1$^{+}$ 290; nmr δ ($CDCl_3$) 1.45(s,9H), 3.67(s,3H), 3.73(s,3H).

c) N-(2-(1,1-Dimethylethoxy)-2-oxoethyl-D-norvaline methyl ester

nmr δ ($CDCl_3$) 1.46(s,9H) and 3.72(s,3H)

d) 2-(3-(Acetylthio)-2(S)-methyl-1-oxopropyl-1-((1,1,dimethylethoxy)carbonyl)hydrazineacetic acid methyl ester

Using methyl bromoacetate and cesium carbonate.

M + 1$^{+}$ 349; nmr δ ($CDCl_3$) 1.46(s,9H), 2.34(s,3H)

e) N-(3-(Acetylthio)-2(S)-methyl-1-oxopropyl)-N-(2-methoxy-2-oxoethoxyl)glycine 1,1-dimethylethyl ester

Using 1,1-dimethylethyl bromoacetate and potassium iodide catalyst on the product of Example H.

M + 1$^{+}$ 364; nmr δ ($CDCl_3$) 1.46(s,9H), 2.33(s,3H), 3.77(s,3H)

f) N-(4-(2-methoxy-2-oxoethoxy)phenyl)glycine 1,1-dimethylethyl ester

M$^{+}$ 295; nmr δ ($CDCl_3$) 1,47(s,9H), 3.79(s,3H)

g) (R)-3-((2-(1,1-Dimethylethoxy)-2-oxoethyl)amino)butanoic acid methyl ester

$M+1^+$ 232; nmr δ ($CDCl_3$) 1.47(s,9H), 3.67(s,3H)

h) N-(2-(1,1-Dimethylethoxy)-2-oxoethyl)-L-glutamic acid dimethylester

Example X

In vitro Assay of inhibitors of Angiotensin Converting Enzyme.

The method is based upon that of Cushman and Cheung (1971) but uses a radioactive substrate [glycine-1-$^{14}$C] hippuryl-L-histidyl-L-leucine (HHL) whose hydrolysis may be determined by liquid scintillation counting of released [$^{14}$C]-hippuric acid. Hydrolysis if 2mM HHL by an extract of rabbit lung acetone powder (Sigma) over a 30 minute incubation period at 37° is followed by acidification of the reaction mixture and extraction of [$^{14}$C]hippurate with ethyl acetate.

Potential inhibitors are tested initially at 0.01mM and if found active are retested at lower concentrations to determine an $IC_{50}$ or other kinetic parameters. Dimethyl sulphoxide at 1% final concentration may be used as a solubility aid without affecting enzyme activity. Compounds of special interest are studied at a range of substrate and inhibitor concentrations to determine the type of inhibition and are also tested against other enzymes, eg carboxpeptidase A to establish their specificity for ACE.

Example Y

Duration of action is investigated in anaesthetised male Sprague-Dawley rats. Arterial blood pressure and heart rate are measured by direct catheterisation, and pressure responses to angiotensin I are measured at 5 minute intervals. Percentage decreases in the pressure responses to angiotensin I due to infusion of the short acting ACE inhibitor are measured, followed by calculation of the rate of recovery after cessation of infusion.

**Claims**

1.A compound of formula I,

$ZCHR_1COR_2$ I

in which $R_2$ is a group of formula II,

$-NR_xCHR_yCOOH$ II

in which $R_x$ and $R_y$, which may be the same or different, are each cycloalkyl C3 to 12, straight chain or branched chain alkyl C 1 to 6 optionally substituted by phenyl; phenylalkyl C7 to 12, phenyl, phenyloxyalkyl, N-alkyl, O-alkyl, or together $R_x$ and $R_y$ may form a heterocyclic group optionally substituted by cycloalkyl C 3 to 12, straight chain or branched chain alkyl C 1 to 6 phenyl; phenylalkyl C7 to 12, phenyl, phenyloxyalkyl, N-alkyl, O-alkyl, or one of $R_x$ and $R_y$ may be hydrogen,
provided that one of $R_x$ and $R_y$, the heterocyclic group formed by $R_x$ and $R_y$ or the optional substituents is substituted by one or two groups $-COOR_9$
$R_9$ is phenyl, $-(CH_2)_xCONR_{14}R_{15}$, $-(CH_2)_yCOOR_{16}$ or straight, branched or cyclic alkyl C 1 to 12 optionally substituted by phenyl;
Z is $R_{20}$ $CH(COOH)NR_{21}$, $R_{26}SCH_2-$, $R_{22}$ $\underset{O}{\underset{\|}{P}}(OH)-$,

or $R_{23}CONR_{24}CHR_{25}$ $CO(CH_2)_z-$,
$R_1$, $R_{14}$, $R_{15}$, $R_{21}$ and $R_{24}$, which may be the same or different, are each hydrogen or alkyl C 1 to 6,
$R_{16}$ is alkyl C 1 to 6,
$R_{20}$, $R_{22}$, $R_{23}$ and $R_{25}$, which may be the same or different, are each phenyl or phenylalkyl C 7 to 12,
$R_{26}$ is hydrogen or alkanoyl C2 to 7,
x, y and z, which may be the same or different, are each an integer from 1 to 6,
and pharmaceutically acceptable salts thereof.

2. A compound according to claim 1 wherein $R_x$ is a group $R_3$ or $R_5$ and $R_y$ is a group $R_4$ or $(CH_2)_nCOOR_6$
or in which $R_x$ and $R_y$ together form a group of formula XXVII

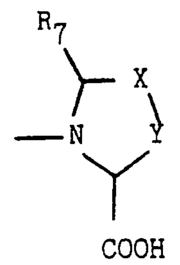

XXVII

in which one of X and Y is $-CHR_8$ or S and the remainder is $-CH_2-$
$R_3$ is cycloalkyl C 3 to 12, straight chain or branched chain alkyl C 1 to 6 optionally substituted by phenyl; phenylalkyl C7 to 12, phenyl, phenyloxyalkyl, N-alkyl or O-alkyl; each of which is substituted by one or two groups $-COOR_9$,
$R_5$ is hydrogen, cycloalkyl C3 to 12 or straight chain or branched chain alkyl C 1 to 7
$R_6$ is alkyl C 1 to 6, $-(CH_2)_vCOOR_{10}$ or $(CH_2)_wCONR_{11}R_{12}$,
one of $R_7$ and $R_8$ is $-A(CH_2)_mCOOR_{13}$ and the remainder is hydrogen or phenyl,
$R_{13}$ is alkyl C 1 to 6, $-CH_2COOR_{17}$ or $CH_2CONR_{18}R_{19}$,
$R_4$, $R_{11}$, $R_{12}$, $R_{18}$ and $R_{19}$, which may be the same or different, are each hydrogen or alkyl C 1 to 6,
$R_{10}$ and $R_{17}$, which may be the same or different, are each alkyl C 1 to 6,
n, v and w which may be the same or different, are each an integer from 1 to 6,
m is an integer from 0 to 6,
A is -O- or a bond, and
$R_9$ is as defined in claim 1.

3. A compound according to Claim 2, comprising compounds of formula XXI
$ZCHR_1CON(CH_2COOH)(CH_2)_tCOOR_9$ XXI
in which Z, $R_1$ and $R_9$ are as defined in claim 2, and
t is an integer from 1 to 6.

4. A compound according to Claim 2 comprising compounds of formula XXII.
$ZCHR_1CON(CH_2COOH)CH(COOR_9)R_{27}$ XXII
in which Z, $R_1$ and $R_9$ are as defined in claim 2, and
$R_{27}$ is cycloalkyl C 3 to 12, straight chain or branched chain alkyl C 1 to 6, preferably alkyl C 1 to 4.

5. A compound according to Claim 2 wherein $ZCHR_1CO$ is $R_{26}SCH_2(CH_3)CO$ and $R_{26}$ is as defined in Claim 1.

6. N-(3-(Acetylthio)-2(S)-methyl-1-oxopropyl)-N-(carboxymethyl)-β-alanine 1-methyl ester,
N-(Carboxymethyl)-N-(3-mercapto-2(S)-methyl-1-oxopropyl)-β-alanine 1-methyl ester dicyclohexylamine salt,
N-(3-Mercapto-2(S)-methyl-1-oxopropyl)-N-(2-methoxy-2-oxoethyl)glycine dicyclohexylamine salt,
3-((3-(Acetylthio)-2(S)-methyl-1-oxopropyl)(carboxymethyl)amino)benzoic acid 1-methyl ester,
3-((Carboxymethyl)(3-mercapto-2(S)-methyl-1-oxopropyl)amino)benzoic acid 1-methylester,
N-(3-(Acetylthio)-2(S)-methyl-1-oxopropyl)-N- (carboxymethyl)-β-alanine 2-(dimethylamino)-2-oxoethyl ester,
N-(Carboxymethyl)-N-(3-mercapto-2(S)-methyl-1-oxopropyl)-β-alanine 1-(2-(dimethylamino)-2-oxoethyl) ester dicyclohexylamine salt,
N-(5-(Benzoylamino)-1,4-dioxo-6-phenylhexyl)-N-(carboxymethyl)-β-alanine 1-(2-(dimethylamino)-2-oxoethyl) ester dicyclohexylamine salt,
N-(5-(Benzoylamino)-1,4-dioxo-6-phenylhexyl)-N-(2-oxo-2-(phenylmethoxy)ethyl)glycine dicyclohexylamine salt,
N-(5-(Benzoylamino)-1,4-dioxo-6-phenylhexyl)-N-(2-oxo-2-phenoxyethyl)glycine dicyclohexylamine salt,
S-2-((2-((Carboxymethyl)(3-methoxy-3-oxopropyl)amino)-1(S)-methyl-2-oxoethyl)amino)benzenebutanoic acid trifluoroacetate salt,
trans-1-(3-(Acetylthio)-2(S)-methyl-1-oxopropyl)-4-(2-ethoxy-2-oxoethoxy)-L-proline,
trans-4-(2-Ethoxy-2-oxoethoxy)-1-(3-mercapto-2(S)-methyl-1-oxopropyl)-L-proline dicyclohexylamine salt,
trans-1-(N-(1(S)-Carboxy-3-phenylpropyl)-L-alanyl)-4-(2-ethoxy-2-oxoethoxy)-L-proline,
N-(3-(Acetylthio)-2(S)-methyl-1-oxopropyl)-L-glutamic acid 5-methyl ester,
N-(3-Mercapto-2(S)-methyl-1-oxopropyl)-L-glutamic acid 5-methylester dicyclohexylamine salt,
N-(3-Mercapto-2(S)-methyl-1-oxopropyl)-L-aspartic acid 4-methylester dicyclohexylamine salt,
(2S-cis)-3-(3-(Acetylthio)-2(S)-methyl-1-oxopropyl)-2,4-thiazolidinedicarboxylic acid 4-methyl ester,
(2S-cis)-3-(3-Mercapto-2(S)-methyl-1-oxopropyl)-2,4-thiazolidinedicarboxylic acid 4-methyl ester dicyclohexylamine salt,
trans-4-(2-Ethoxy-2-oxoethoxy)-1-((hydroxy(4-phenylbutyl)phosphinyl)acetyl)-L-proline 1:2 salt with adamantanamine,
N-(Carboxymethyl)-N-((hydroxy(4-phenylbutyl)phosphinyl)acetyl)-β-alanine 1-methyl ester,
N-(3-(Acetylthio)-2(S)-methyl-1-oxopropyl)-N-cyclopentyl)-L-glutamic acid 5-methyl ester,
3-((3-(Acetylthio)-2(S)-methyl-1-oxopropyl)(carboxymethyl)amino)pentanedioic acid 1,5 -dimethyl ester,
(S-(2α,4α,5α)-1-(3-(Acetylthio)-2(S)-methyl-1-oxopropyl)-5-phenyl-2,4-pyrrolidinedicarboxylic acid 4-methyl ester,
2-((3-(Acetylthio)-2(S)-methyl-1-oxopropyl)(carboxymethyl)amino)benzoic acid 1-methyl ester,
4-((3-(Acetylthio)-2(S)-methyl-1-oxopropyl)(carboxymethyl)amino)benzeneacetic acid 1-methyl ester,
4-((3-(Acetylthio)-2(S)-methyl-1-oxopropyl)(carboxymethyl)amino)benzenepropanoic acid 1-methyl ester,
N-(3-(Acetylthio)-2(S)-methyl-1-oxopropyl)-N-(carboxymethyl)-D-alanine 1-methyl ester,
N-(3-(Acetylthio)-2(S)-methyl-1-oxopropyl)-N-(carboxymethyl)-L-alanine 1-methyl ester,
2-((3-(Acetylthio)-2(S)-methyl-1-oxopropyl) (carboxymethyl)amino)-2-methylpropanoic acid 1-methyl ester,
4-((3-(Acetylthio)-2(S)-methyl-1-oxopropyl)(carboxymethyl)amino)butanoic acid 1-methyl ester,
N-(3-Acetylthio)-2(S)-methyl-1-oxopropyl)-N-(carboxymethyl)-β-alanine 1-phenyl ester,
N-(5-(Benzoylamino)-1,4-dioxo-6-phenylhexyl)-N-(carboxymethyl)-β-alanine 1-methyl ester dicyclohexylamine salt,
(R)-2-((3-(Acetylthio)-2(S)-methyl-1-oxopropyl)(carboxymethyl)amino)butanoic acid 1-methyl ester,
N-(3-(Acetylthio)-2(S)-methyl-1-oxopropyl)-N-(carboxymethyl)-D-leucine 1-methyl ester,
N-(3-(Acetylthio)-2(S)-methyl-1-oxopropyl)-N-(carboxymethyl)-D-valine 1-methyl ester,
N-(3-(Acetylthio)-2(S)-methyl-1-oxopropyl)-N-(carboxymethyl)-D-glutamic acid 1,5-dimethyl ester,
N-(3-(Acetylthio)-2(S)-methyl-1-oxopropyl)-N-(2-methoxy-2-oxoethyl)-L-alanine,
N-(3-(Acetylthio)-2(S)-methyl-1-oxopropyl)-N- (carboxymethyl)-D-alanine 2-methylpropyl ester,
5-((3-(Acetylthio)-2(S)-methyl-1-oxopropyl)(carboxymethyl)amino)pentanoic acid 1-methyl ester,
N-(3-(Acetylthio)-2(S)-methyl-1-oxopropyl)-N-(carboxymethyl)-D-norvaline 1-methyl ester,
N-(3-(Acetylthio)-2(S)-methyl-1-oxopropyl)-N-(carboxymethyl)-D-phenylalanine 1-methyl ester,
1-(3-(Acetylthio)-2(S)-methyl-1-oxopropyl)-1,2-hydrazinediacetic acid 2-methyl ester,
N-(3-(Acetylthio)-2(S)-methyl-1-oxopropyl)-N-(2-methoxy-2-oxoethoxy)glycine,
4-((3-(Acetylthio)-2(S)-methyl-1-oxopropyl)(carboxymethyl)amino)benzoic acid 1-methyl ester,
N-(3-(Acetylthio)-2(S)-methyl-1-oxopropyl)-N-(4-(2-methoxy-2-oxoethoxy)phenyl)glycine,
(2R-cis)-1-(3-(Acetylthio)-2(S)-methyl-1-oxopropyl)-5-carboxy-2-pyrrolidineacetic acid 2-methyl ester,
N-(3-(Acetylthio)-2(S)-methyl-1-oxopropyl)-N-(carboxymethyl)-β-alanine 2-methylpropyl ester,
N-(3-(Acetylthio)-2(S)-methyl-1-oxopropyl)-N-(carboxymethyl)-β-alanine ethyl ester,
4-((3-(Acetylthio)-2(S)-methyl-1-oxopropyl)(carboxymethyl)amino)butanoic acid 1-(2-methylpropyl)ester,
(R)-3-((3-(Acetylthio)-2(S)-methyl-1-oxopropyl)(carboxymethyl)amino)butanoic acid 1-methyl ester,
N-(3-(Acetylthio)-2(S)-methyl-1-oxopropyl)-N-carboxymethyl)-L-glutamic acid 1,5-dimethyl ester,
N-(3-(Acetylthio)-2(S)-methyl-1-oxopropyl)-N-(2-methoxy-2-oxoethyl)glycine,
2-((Carboxymethyl)(3-mercapto-2(S)-methyl-1-oxopropyl)amino)benzoic acid 1-methyl ester dicyclohexylamine salt,
4-((Carboxymethyl)(3-mercapto-2(S)-methyl-1-oxopropyl)amino)benzeneacetic acid 1-methyl ester dicyclohexylamine salt,
4-((Carboxymethyl)(3-mercapto-2(S)-methyl-1-oxopropyl)amino)benzenepropanoic acid 1-methyl ester dicyclohexylamine salt,
N-(Carboxymethyl)-N-(3-mercapto-2(S)-methyl-1-oxopropyl)-D-alanine 1-methyl ester dicyclohexylamine salt,
N-(Carboxymethyl)-N-(3-mercapto-2(S)-methyl-1-oxopropyl)-L-alanine 1-methyl ester dicyclohexylamine salt,
2-((Carboxymethyl)(3-mercapto-2(S)-methyl-1-oxopropyl)amino)-2-methylpropanoic acid 1-methyl ester dicyclohexylamine salt,
4-((Carboxymethyl)(3-mercapto-2(S)-methyl-1-oxopropyl)amino)butanoic acid 1-methyl ester 1-adamantanamine salt,
N-(Cyclopentyl)-N-(3-mercapto-2(S)-methyl-1-oxopropyl)-L-glutamic acid 5-methyl ester,
N-(Carboxymethyl)-N-(3-mercapto-2(S)-methyl-1-oxopropyl)-D-leucine 1-methyl ester adamantanamine salt,
3-((Carboxymethyl)(3-mercapto-2(S)-methyl-1-oxopropyl)amino)pentanedioic acid 1,5-dimethyl ester adamantanamine salt,
(S-(2α,4α,5α))-1-(3-mercapto-2(S)-methyl-1-oxopropyl)-5-phenyl-2,4-pyrrolidinedicarboxylic acid 4-methyl ester,
N-(Carboxymethyl)-N-(3-mercapto-2(S)-methyl-1-oxopropyl)-D-valine 1-methyl ester dicyclohexylamine salt,

N-(Carboxymethyl)-N-(3-mercapto-2(S)-methyl-1-oxopropyl)-D-glutamic acid 1,5-dimethyl ester adamantanamine salt,
(2α,4α,5α)-1-(3-Mercapto-1-oxopropyl)-5-phenyl-2,4-pyrrolidinedicarboxylic acid 4-methyl ester,
N-(3-(Mercapto-2(S)-methyl-1-oxopropyl)-N-(2-methoxy-2-oxoethyl)-L-alanine dicyclohexylamine salt,
N-(Carboxymethyl)-N-(3-mercapto-2(S)-methyl-1-oxopropyl)-D-alanine 2-methylpropyl ester adamantanamine salt,
5-((Carboxymethyl)(3-mercapto-2(S)-methyl-1-oxopropyl)amino)pentanoic acid 1-methyl ester adamantanamine salt,
N-(Carboxymethyl)-N-(3-mercapto-2(S)-methyl-1-oxopropyl)-D-norvaline 1-methyl ester dicyclohexylamine salt,
N-(Carboxymethyl)-N-(3-mercapto-2(S)-methyl-1-oxopropyl)-D-phenylalanine 1-methyl ester adamantanamine salt,
N-(Carboxymethyl)-N-(3-mercapto-2(S)-methyl-1-oxopropyl)-β-alanine 1-ethyl ester adamantanamine salt,
N-(Carboxymethyl)-N-(3-mercapto-2(S)-methyl-1-oxopropyl)-L-glutamic acid 1,5-dimethyl ester adamantanamine salt
N-Carboxymethyl)-N-(3-mercapto-2(S)-methyl-1-oxopropyl)-β-alanine 1-(2-cyclohexylethyl) ester adamantanamine salt
N-(Carboxymethyl)-N-(3-mercapto-2(S)-methyl-1-oxopropyl)-β-alanine 1-cyclohexyl ester adamantanamine salt
(R)-3-((Carboxymethyl)(3-mercapto-2(S)-methyl-1-oxopropyl)amino)butanoic acid 1-methyl ester adamantanamine salt
4-((Carboxymethyl)(3-mercapto-2(S)-methyl-1-oxopropyl)amino)butanoic acid 1-(2-methylpropyl) ester adamantanamine salt
N-(Carboxymethyl)-N-(3-mercapto-2(S)-methyl-1-oxopropyl)-β-alanine 1-(2-methylpropyl) ester adamantanamine salt
N-(Carboxymethyl)-N-(3-mercapto-2(S)-methyl-1-oxopropyl)-β-alanine 1-phenyl ester dicyclohexylamine salt,
(R)-2-((Carboxymethyl)(3-mercapto-2(S)-methyl-1-oxopropyl)amino)butanoic acid 1-methyl ester dicyclohexylamine salt,
N-(2-(Acetylthio)-2(S)-methyl-1-oxopropyl)-N- (carboxymethyl)-β-alanine 1-cyclohexyl ester
N-(3-(Acetylthio)-2(S)-methyl-1-oxopropyl)-N-(carboxymethyl)-β-alanine 1-(2-cyclohexylethyl) ester
(2S-trans)-1-(3-(Acetylthio)-2(S)-methyl-1-oxopropyl)-2,4-pyrrolidinedicarboxylic acid 4-methyl ester
(2R-cis)-3-(3-(Acetylthio)-2(S)-methyl-1-oxopropyl)-2,4-thiazolidinedicarboxylic acid 2-methyl ester,
and pharmaceutically acceptable salts, thereof.

7. A compound of formula I according to Claim 1 for use as a medicament.

8. A pharmaceutical composition comprising preferably less than 80% by weight of a compound of formula I, or a pharmaceutically acceptable salt thereof, in admixture with a pharmaceutically acceptable adjuvant, diluent or carrier.

9. A process for the production of a compound according to Claim 1, or a pharmaceutically acceptable salt thereof which comprises;
a) removal of a protecting group from a compound of formula I in which one or more of the amino, thiol or carboxylic acid groups is protected,
b) reacting a compound of formula III, or a salt or ester thereof,
$ZCHR_1COL_b$ III
in which Z and $R_1$ are as defined in claim 1, and
$L_b$ is a good leaving group or -OH
with a compound of formula IV, or a salt or ester thereof,
$HNR_xCHR_yCOOH$ IV
in which $R_x$ and $R_y$ are as defined in claim 1,
c) production of a pharmaceutically acceptable salt of a compound of formula I, by treating a compound of formula I, or another salt thereof, with a compound containing an available pharmaceutically acceptable ion and capable of converting the compound of formula I or the other salt thereof, to a pharmaceutically acceptable salt of the compound of formula I,
and where desired or necessary deprotecting the resulting compound, or converting a compound of formula I to a pharmaceutically acceptable salt thereof or vice versa.

10. Compounds of formula IV
$HNR_xCHR_yCOOH$ IV
in which $R_x$ and $R_y$ are as defined in claim 1, for use as intermediates.

Claims for the following Contracting States: GR, ES

1.A process for the preparation of a compound of formula I,

$ZCHR_1COR_2$ I

in which $R_2$ is a group of formula II,

$-NR_xCHR_yCOOH$ II

in which $R_x$ and $R_y$, which may be the same or different, are each cycloalkyl C3 to 12, straight chain or branched chain alkyl C 1 to 6 optionally substituted by phenyl; phenylalkyl C7 to 12, phenyl, phenyloxyalkyl, N-alkyl, O-alkyl, or together $R_x$ and $R_y$ may form a heterocyclic group optionally substituted by cycloalkyl C 3 to 12, straight chain or branched chain alkyl C 1 to 6 phenyl; phenylalkyl C7 to 12, phenyl, phenyloxyalkyl, N-alkyl, O-alkyl, or one of $R_x$ and $R_y$ may be hydrogen,

provided that one of $R_x$ and $R_y$, the heterocyclic group formed by $R_x$ and $R_y$ or the optional substituents is substituted by one or two groups $-COOR_9$

$R_9$ is phenyl, $-(CH_2)_xCONR_{14}R_{15}$, $-(CH_2)_yCOOR_{16}$ or straight, branched or cyclic alkyl C 1 to 12 optionally substituted by phenyl;

Z is $R_{20}$ $CH(COOH)NR_{21}$, $R_{26}SCH_2$-, $R_{22}\underset{\overset{\|}{O}}{P}(OH)$-,

or $R_{23}CONR_{24}CHR_{25}$ $CO(CH_2)_z$-,

$R_1$, $R_{14}$, $R_{15}$, $R_{21}$ and $R_{24}$, which may be the same or different, are each hydrogen or alkyl C 1 to 6,

$R_{16}$ is alkyl C 1 to 6,

$R_{20}$, $R_{22}$, $R_{23}$ and $R_{25}$, which may be the same or different, are each phenyl or phenylalkyl C 7 to 12,

$R_{26}$ is hydrogen or alkanoyl C2 to 7,

x, y and z, which may be the same or different, are each an integer from 1 to 6,

and pharmaceutically acceptable salts thereof, which comprises;

a) removal of a protecting group from a compound of formula I in which one or more of the amino, thiol or carboxylic acid groups is protected,

b) reacting a compound of formula III, or a salt or ester thereof,

$ZCHR_1COL_b$ III

in which Z and $R_1$ are as defined in claim 1, and

$L_b$ is a good leaving group or -OH

with a compound of formula IV, or a salt or ester thereof,

$HNR_xCHR_yCOOH$ IV

in which $R_x$and $R_y$ are as defined in claim 1,

c) production of a pharmaceutically acceptable salt of a compound of formula I, by treating a compound of formula I, or another salt thereof, with a compound containing an available pharmaceutically acceptable ion and capable of converting the compound of formula I or the other salt thereof, to a pharmaceutically acceptable salt of the compound of formula I,

and where desired or necessary deprotecting the resulting compound, or converting a compound of formula I to a pharmaceutically acceptable salt thereof or vice versa.

2. A process according to claim 1 wherein $R_x$ is a group $R_3$ or $R_5$ and $R_y$ is a group $R_4$ or $(CH_2)_nCOOR_6$

or in which $R_x$ and $R_y$ together form a group of formula XXVII

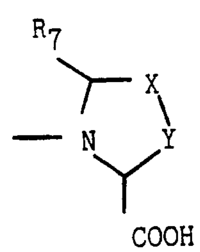

XXVII

in which one of X and Y is $-CHR_8$ or S and the remainder is $-CH_2$-

$R_3$ is cycloalkyl C 3 to 12, straight chain or branched chain alkyl C 1 to 6 optionally substituted by phenyl; phenylalkyl C7 to 12, phenyl, phenyloxyalkyl, N-alkyl or O-alkyl; each of which is substituted by one or two groups $-COOR_9$,

$R_5$ is hydrogen, cycloalkyl C3 to 12 or straight chain or branched chain alkyl C 1 to 7

$R_6$ is alkyl C 1 to 6, $-(CH_2)_vCOOR_{10}$ or $(CH_2)_wCONR_{11}R_{12}$,

one of $R_7$ and $R_8$ is $-A(CH_2)_mCOOR_{13}$ and the remainder is hydrogen or phenyl,
$R_{13}$ is alkyl C 1 to 6, $-CH_2COOR_{17}$ or $CH_2CONR_{18}R_{19}$,
$R_4$, $R_{11}$, $R_{12}$, $R_{18}$ and $R_{19}$, which may be the same or different, are each hydrogen or alkyl C 1 to 6,
$R_{10}$ and $R_{17}$, which may be the same or different, are each alkyl C 1 to 6,
n, v and w which may be the same or different, are each an integer from 1 to 6,
m is an integer from 0 to 6,
A is -O- or a bond, and
$R_9$ is as defined in claim 1.

3. A process according to Claim 2, comprising compounds of formula XXI
$ZCHR_1CON(CH_2COOH)(CH_2)_tCOOR_9$ XXI
in which Z, $R_1$ and $R_9$ are as defined in claim 2, and
t is an integer from 1 to 6.

4. A process according to Claim 2 comprising compounds of formula XXII,
$ZCHR_1CON(CH_2COOH)CH(COOR_9)R_{27}$ XXII
in which Z, $R_1$ and $R_9$ are as defined in claim 2, and
$R_{27}$ is cycloalkyl C 3 to 12, straight chain or branched chain alkyl C 1 to 6, preferably alkyl C 1 to 4.

5. A process according to Claim 2 wherein $ZCHR_1CO$ is $R_{26}SCH_2(CH_3)CO$ and $R_{26}$ is as defined in Claim 1.

6. A process according to Claim 5 wherein $R_{26}$ is hydrogen.

7. A process according to Claim 1 wherein the compound is
N-(3-(Acetylthio)-2(S)-methyl-1-oxopropyl)-N-(carboxymethyl)-β-alanine 1-methyl ester,
N-(Carboxymethyl)-N-(3-mercapto-2(S)-methyl-1-oxopropyl)-β-alanine 1-methyl ester dicyclohexylamine salt,
N-(3-Mercapto-2(S)-methyl-1-oxopropyl)-N-(2-methoxy-2-oxoethyl)glycine dicyclohexylamine salt,
3-((3-(Acetylthio)-2(S)-methyl-1-oxopropyl)(carboxymethyl)amino)benzoic acid 1-methyl ester,
3-((Carboxymethyl)(3-mercapto-2(S)-methyl-1 oxopropyl)amino)benzoic acid 1-methylester,
N-(3-(Acetylthio)-2(S)-methyl-1-oxopropyl)-N-(carboxymethyl)-β-alanine 2-(dimethylamino)-2-oxoethyl ester,
N-(Carboxymethyl)-N-(3-mercapto-2(S)-methyl-1-oxopropyl)-β-alanine 1-(2-(dimethylamino)-2-oxoethyl) ester dicyclohexylamine salt,
N-(5-(Benzoylamino)-1,4-dioxo-6-phenylhexyl)-N-(carboxymethyl)-β-alanine 1-(2-(dimethylamino)-2-oxoethyl)ester dicyclohexylamine salt,
N-(5-(Benzoylamino)-1,4-dioxo-6-phenylhexyl)-N-(2-oxo-2-(phenylmethoxy)ethyl)glycine dicyclohexylamine salt,
N-(5-(Benzoylamino)-1,4-dioxo-6-phenylhexyl)-N-(2-oxo-2-phenoxyethyl)glycine dicyclohexylamine salt,
S-2-((2-((Carboxymethyl)(3-methoxy-3-oxopropyl)amino)-1(S)-methyl-2-oxoethyl)amino)benzenebutanoic acid trifluoroacetate salt,
trans-1-(3-(Acetylthio)-2(S)-methyl-1-oxopropyl)-4-(2-ethoxy-2-oxoethoxy)-L-proline,
trans-4-(2-Ethoxy-2-oxoethoxy)-1-(3-mercapto-2(S)- methyl-1-oxopropyl)-L-proline dicyclohexylamine salt,
trans-1-(N-(1(S)-Carboxy-3-phenylpropyl)-L-alanyl)-4-(2-ethoxy-2-oxoethoxy)-L-proline,
N-(3-(Acetylthio)-2(S)-methyl-1-oxopropyl)-L-glutamic acid 5-methyl ester,
N-(3-Mercapto-2(S)-methyl-1-oxopropyl)-L-glutamic acid 5-methylester dicyclohexylamine salt,
N-(3-Mercapto-2(S)-methyl-1-oxopropyl)-L-aspartic acid 4-methylester dicyclohexylamine salt,
(2S-cis)-3-(3-(Acetylthio)-2(S)-methyl-1-oxopropyl)-2,4-thiazolidinedicarboxylic acid 4-methyl ester,
(2S-cis)-3-(3-Mercapto-2(S)-methyl-1-oxopropyl)-2,4-thiazolidinedicarboxylic acid 4-methyl ester dicyclohexylamine salt,
trans-4-(2-Ethoxy-2-oxoethoxy)-1-((hydroxy(4-phenylbutyl)phosphinyl)acetyl)-L-proline 1:2 salt with adamantanamine,
N-(Carboxymethyl)-N-((hydroxy(4-phenylbutyl)phosphinyl)acetyl)-β-alanine 1-methyl ester,
N-(3-(Acetylthio)-2(S)-methyl-1-oxopropyl)-N-cyclopentyl)-L-glutamic acid 5-methyl ester,
3-((3-(Acetylthio)-2(S)-methyl-1-oxopropyl)(carboxymethyl)amino)pentanedioic acid 1,5 -dimethyl ester,
(S-(2α,4α,5α)-1-(3-(Acetylthio)-2(S)-methyl-1-oxopropyl)-5-phenyl-2,4-pyrrolidinedicarboxylic acid 4-methyl ester,
2-((3-(Acetylthio)-2(S)-methyl-1-oxopropyl)(carboxymethyl)amino)benzoic acid 1-methyl ester,
4-((3-(Acetylthio)-2(S)-methyl-1-oxopropyl)(carboxymethyl)amino)benzeneacetic acid 1-methyl ester,
4-((3-(Acetylthio)-2(S)-methyl-1-oxopropyl)(carboxymethyl)amino)benzenepropanoic acid 1-methyl ester,
N-(3-(Acetylthio)-2(S)-methyl-1-oxopropyl)-N-(carboxymethyl)-D-alanine 1-methyl ester,
N-(3-(Acetylthio)-2(S)-methyl-1-oxopropyl)-N-(carboxymethyl)-L-alanine 1-methyl ester,
2-((3-(Acetylthio)-2(S)-methyl-1-oxopropyl)(carboxymethyl)amino)-2-methylpropanoic acid 1-methyl ester,

4-((3-(Acetylthio)-2(S)-methyl-1-oxopropyl)(carboxymethyl)amino)butanoic acid 1-methyl ester,
N-(3-Acetylthio)-2(S)-methyl-1-oxopropyl)-N-(carboxymethyl)-β-alanine 1-phenyl ester,
N-(5-(Benzoylamino)-1,4-dioxo-6-phenylhexyl)-N-(carboxymethyl)-β-alanine 1-methyl ester dicyclohexylamine salt,
(R)-2-((3-(Acetylthio)-2(S)-methyl-1-oxopropyl)(carboxymethyl)amino)butanoic acid 1-methyl ester,
N-(3-(Acetylthio)-2(S)-methyl-1-oxopropyl)-N-(carboxymethyl)-D-leucine 1-methyl ester,
N-(3-(Acetylthio)-2(S)-methyl-1-oxopropyl)-N-(carboxymethyl)-D-valine 1-methyl ester,
N-(3-(Acetylthio)-2(S)-methyl-1-oxopropyl)-N-(carboxymethyl)-D-glutamic acid 1,5-dimethyl ester,
N-(3-(Acetylthio)-2(S)-methyl-1-oxopropyl)-N-(2-methoxy-2-oxoethyl)-L-alanine,
N-(3-(Acetylthio)-2(S)-methyl-1-oxopropyl)-N-(carboxymethyl)-D-alanine 2-methylpropyl ester,
5-((3-(Acetylthio)-2(S)-methyl-1-oxopropyl)(carboxymethyl)amino)pentanoic acid 1-methyl ester,
N-(3-(Acetylthio)-2(S)-methyl-1-oxopropyl)-N-(carboxymethyl)-D-norvaline 1-methyl ester,
N-(3-(Acetylthio)-2(S)-methyl-1-oxopropyl)-N-(carboxymethyl)-D-phenylalanine 1-methyl ester,
1-(3-(Acetylthio)-2(S)-methyl-1-oxopropyl)-1,2-hydrazinediacetic acid 2-methyl ester,
N-(3-(Acetylthio)-2(S)-methyl-1-oxopropyl)-N-(2-methoxy-2-oxoethoxy)glycine,
4-((3-(Acetylthio)-2(S)-methyl-1-oxopropyl)(carboxymethyl)amino)benzoic acid 1-methyl ester,
N-(3-(Acetylthio)-2(S)-methyl-1-oxopropyl)-N-(4-(2-methoxy-2-oxoethoxy)phenyl)glycine,
(2R-cis)-1-(3-(Acetylthio)-2(S)-methyl-1-oxopropyl)-5-carboxy-2-pyrrolidineacetic acid 2-methyl ester,
N-(3-(Acetylthio)-2(S)-methyl-1-oxopropyl)-N- (carboxymethyl)-β-alanine 2-methylpropyl ester,
N-(3-(Acetylthio)-2(S)-methyl-1-oxopropyl)-N-(carboxymethyl)-β-alanine ethyl ester,
4-((3-(Acetylthio)-2(S)-methyl-1-oxopropyl)(carboxymethyl)amino)butanoic acid 1-(2-methylpropyl)ester,
(R)-3-((3-(Acetylthio)-2(S)-methyl-1-oxopropyl)(carboxymethyl)amino)butanoic acid 1-methyl ester,
N-(3-(Acetylthio)-2(S)-methyl-1-oxopropyl)-N-carboxymethyl)-L-glutamic acid 1,5-dimethyl ester,
N-(3-(Acetylthio)-2(S)-methyl-1-oxopropyl)-N-(2-methoxy-2-oxoethyl)glycine,
2-((Carboxymethyl)(3-mercapto-2(S)-methyl-1-oxopropyl)amino)benzoic acid 1-methyl ester dicyclohexylamine salt,
4-((Carboxymethyl)(3-mercapto-2(S)-methyl-1-oxopropyl)amino)benzeneacetic acid 1-methyl ester dicyclohexylamine salt,
4-((Carboxymethyl)(3-mercapto-2(S)-methyl-1-oxopropyl)amino)benzenepropanoic acid 1-methyl ester dicyclohexylamine salt,
N-(Carboxymethyl)-N-(3-mercapto-2(S)-methyl-1-oxopropyl)-D-alanine 1-methyl ester dicyclohexylamine salt,
N-(Carboxymethyl)-N-(3-mercapto-2(S)-methyl-1-oxopropyl)-L-alanine 1-methyl ester dicyclohexylamine salt,
2-((Carboxymethyl)(3-mercapto-2(S)-methyl-1-oxopropyl)amino)-2-methylpropanoic acid 1-methyl ester dicyclohexylamine salt,
4-((Carboxymethyl)(3-mercapto-2(S)-methyl-1-oxopropyl)amino)butanoic acid 1-methyl ester 1-adamantanamine salt,
N-(Cyclopentyl)-N-(3-mercapto-2(S)-methyl-1-oxopropyl)-L-glutamic acid 5-methyl ester,
N-(Carboxymethyl)-N-(3-mercapto-2(S)-methyl-1-oxopropyl)-D-leucine 1-methyl ester adamantanamine salt,
3-((Carboxymethyl)(3-mercapto-2(S)-methyl-1-oxopropyl)amino)pentanedioic acid 1,5-dimethyl ester adamantanamine salt,
(S-(2α,4α,5α))-1-(3-mercapto-2(S)-methyl-1-oxopropyl)-5-phenyl-2,4-pyrrolidinedicarboxylic acid 4-methyl ester,
N-(Carboxymethyl)-N-(3-mercapto-2(S)-methyl-1-oxopropyl)-D-valine 1-methyl ester dicyclohexylamine salt,
N-(Carboxymethyl)-N-(3-mercapto-2(S)-methyl-1-oxopropyl)-D-glutamic acid 1,5-dimethyl ester adamantanamine salt,
(2α,4α,5α)-1-(3-Mercapto-1-oxopropyl)-5-phenyl-2,4-pyrrolidinedicarboxylic acid 4-methyl ester,
N-(3-(Mercapto-2(S)-methyl-1-oxopropyl)-N-(2-methoxy-2-oxoethyl)-L-alanine dicyclohexylamine salt,
N-(Carboxymethyl)-N-(3-mercapto-2(S)-methyl-1-oxopropyl)-D-alanine 2-methylpropyl ester adamantanamine salt,
5-((Carboxymethyl)(3-mercapto-2(S)-methyl-1- oxopropyl)amino)pentanoic acid 1-methyl ester adamantanamine salt,
N-(Carboxymethyl)-N-(3-mercapto-2(S)-methyl-1-oxopropyl)-D-norvaline 1-methyl ester dicyclohexylamine salt,
N-(Carboxymethyl)-N-(3-mercapto-2(S)-methyl-1-oxopropyl)-D-phenylalanine 1-methyl ester adamantanamine salt,
N-(Carboxymethyl)-N-(3-mercapto-2(S)-methyl-1-oxopropyl)-β-alanine 1-ethyl ester adamantanamine salt,
N-(Carboxymethyl)-N-(3-mercapto-2(S)-methyl-1-oxopropyl)-L-glutamic acid 1,5-dimethyl ester adamantanamine salt
N-Carboxymethyl)-N-(3-mercapto-2(S)-methyl-1-oxopropyl)-β-alanine 1-(2-cyclohexylethyl) ester adamantanamine salt
N-(Carboxymethyl)-N-(3-mercapto-2(S)-methyl-1-oxopropyl)-β-alanine 1-cyclohexyl ester adamantanamine salt
(R)-3-((Carboxymethyl)(3-mercapto-2(S)-methyl-1-oxopropyl)amino)butanoic acid 1-methyl ester adamantanamine salt
4-((Carboxymethyl)(3-mercapto-2(S)-methyl-1-oxopropyl)amino)butanoic acid 1-(2-methylpropyl) ester adamantanamine salt
N-(Carboxymethyl)-N-(3-mercapto-2(S)-methyl-1-oxopropyl)-β-alanine 1-(2-methylpropyl) ester adamantanamine salt
N-(Carboxymethyl)-N-(3-mercapto-2(S)-methyl-1-oxopropyl)-β-alanine 1-phenyl ester dicyclohexylamine salt,
(R)-2-((Carboxymethyl)(3-mercapto-2(S)-methyl-1-oxopropyl)amino)butanoic acid 1-methyl ester dicyclohexylamine salt,
N-(2-(Acetylthio)-2(S)-methyl-1-oxopropyl)-N-(carboxymethyl)-β-alanine 1-cyclohexyl ester
N-(3-(Acetylthio)-2(S)-methyl-1-oxopropyl)-N-(carboxymethyl)-β-alanine 1-(2-cyclohexylethyl) ester
(2S-trans)-1-(3-(Acetylthio)-2(S)-methyl-1-oxopropyl)-2,4-pyrrolidinedicarboxylic acid 4-methyl ester
(2R-cis)-3-(3-(Acetylthio)-2(S)-methyl-1-oxopropyl)-2,4-thiazolidinedicarboxylic acid 2-methyl ester,
and pharmaceutically acceptable salts, thereof.

8. A process for the preparation of a pharmaceutical composition comprising preferably less than 80% by weight of a compound of formula I, or a pharmaceutically acceptable salt thereof, which comprises mixing a compound of formula I with a pharmaceutically acceptable adjuvant, diluent or carrier.

9. Compounds of formula IV

$HNR_xCHR_yCOOH$ IV

in which $R_x$ and $R_y$ are as defined in claim 1, for use as intermediates.

European Patent Office

# EUROPEAN SEARCH REPORT

Application number EP 90301579.0

## DOCUMENTS CONSIDERED TO BE RELEVANT

| Category | Citation of document with indication, where appropriate, of relevant passages | Relevant to claim | CLASSIFICATION OF THE APPLICATION (Int Cl 5) |
|---|---|---|---|
| X | US - A - 4 661 515 (NEISS et al.) * Claim 1; examples * | 1,5,7, 9,10 | C 07 C 327/04<br>A 61 K 31/265<br>C 07 C 323/60<br>A 61 K 31/095<br>C 07 D 207/09<br>C 07 D 277/04<br>C 07 F 9/09 |
| X | US - A - 4 619 944 (YOUSSEFYEH et al.) * Claims 1-4,9 * | 1,5,7, 9,10 | |
| X | DE - A1 - 3 122 541 (MORTON-NORWICH PRODUCTS INC.) * Claim; abstract; examples * | 1,5,7, 9,10 | |
| A | EP - A1 - 0 054 936 (USV PHARMACEUTICAL CORPORATION) * Claim 1 * | 1,5,7, 9,10 | |
| A | DE - A1 - 2 752 720 (E.R.SQUIBB & SONS) * Claims 1,14,17; examples * | 1,5, 7-10 | **TECHNICAL FIELDS SEARCHED (Int Cl 5)** |
| A | GB - A1 - 2 090 591 (ICI) * Claims 1,7,8; examples * | 1,7,9 | C 07 C 327/00<br>C 07 C 323/00<br>A 61 K 31/00<br>C 07 D 207/00<br>C 07 D 277/00<br>C 07 F 9/00 |
| A | EP - A1 - 0 086 757 (FARMATIS) * Claim 1 * | 1 | |

The present search report has been drawn up for all claims

| Place of search | Date of completion of the search | Examiner |
|---|---|---|
| VIENNA | 25-05-1990 | PUSTERER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document